(19) Europäisches Patentamt
European Patent Office
Office européen des brevets

(11) **EP 3 212 718 B1**

(12) **FASCICULE DE BREVET EUROPEEN**

(45) Date de publication et mention
de la délivrance du brevet:
**28.11.2018 Bulletin 2018/48**

(51) Int Cl.:
***C09D 4/06*** *(2006.01)*    ***C09J 4/06*** *(2006.01)*
***C08G 63/676*** *(2006.01)*

(21) Numéro de dépôt: **15788173.1**

(86) Numéro de dépôt international:
**PCT/FR2015/052755**

(22) Date de dépôt: **13.10.2015**

(87) Numéro de publication internationale:
**WO 2016/066918 (06.05.2016 Gazette 2016/18)**

(54) **PRODUITS ETHER-ESTERS ACRYLES MULTIFONCTIONNELS MODIFIES PAR ANHYDRIDE CARBOXILIQUE OU SA FORME POLYACIDE, PROCEDE DE PREPARATION ET COMPOSITIONS RETICULABLES LIEES**

MULTIFUNKTIONELLE, MIT CARBONSÄUREANYDRID ODER DESSEN POLYSÄUREFORM MODIFIZIERTE ACRYL-ETHER-ESTER-PRODUKTE, VERFAHREN ZUR HERSTELLUNG DAVON UND ZUGEHÖRIGE VERNETZBARE ZUSAMMENSETZUNGEN

MULTIFUNCTIONAL ACRYLIC ETHER-ESTER PRODUCTS MODIFIED WITH CARBOXYLIC ANHYDRIDE OR ITS POLYACID FORM, PROCESS FOR PREPARING SAME AND ASSOCIATED CROSSLINKABLE COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.10.2014 FR 1460384**

(43) Date de publication de la demande:
**06.09.2017 Bulletin 2017/36**

(73) Titulaire: **Arkema France**
**92700 Colombes (FR)**

(72) Inventeurs:
• **MONNIER, Guillaume**
**60190 Avrigny (FR)**
• **CICERON, Philippe**
**60300 Senlis (FR)**

• **LEROY, Catherine**
**59800 Lille (FR)**
• **BOURROUSSE, Charles**
**75002 Paris (FR)**

(74) Mandataire: **Schaefer, Anne-Sophie et al**
**ARKEMA France**
**Département Propriété Industrielle**
**420, rue d'Estienne d'Orves**
**92705 Colombes Cedex (FR)**

(56) Documents cités:
**FR-A1- 2 401 946    US-A- 5 096 938**

• **DATABASE WPI Week 201012 Thomson Scientific, London, GB; AN 2010-B33885 XP002741877, & JP 2010 024380 A (TOA GOSEI CHEM IND LTD) 4 février 2010 (2010-02-04) cité dans la demande**

**EP 3 212 718 B1**

**Description**

La présente invention concerne des nouveaux monomères acrylés multifonctionnels qui sont des produits oligoéther-esters acrylés à base d'un mélange de produits acrylés multifonctionnels dérivés de la réaction de l'acide acrylique en défaut avec un polyol multifonctionnel en présence d'un anhydride ou polyacide carboxylique en proportions en équivalents carboxy minoritaires, par rapport à l'acide acrylique, un procédé de préparation, des compositions réticulables à base desdits produits, utilisations desdits produits acrylés comme liants acrylés multifonctionnels de fonctionnalité élevée pour des compositions réticulables de haute densité de réticulation et à faible retrait et plus particulièrement pour des compositions de revêtements pigmentés ou non pigmentés, en particulier des peintures, vernis, encres, adhésifs ou des compositions de moulage, d'étanchéité ou de composite ou de scellement chimique, d'impression 3D, d'objets 3D couche par couche et les produits finis réticulés correspondants.

Des monomères acrylés multifonctionnels de fonctionnalité élevée, d'au moins 3 et pouvant aller jusqu'à 6, en groupements acrylates existent déjà et sont utilisés dans des applications de revêtements comme les vernis ou les encres pour augmenter la densité de réticulation et les performances liées à cette augmentation comme la résistance chimique ou la dureté.

Cependant, les monomères multifonctionnels acrylés existants conduisent à une mauvaise flexibilité, en particulier pour l'application dans les revêtements, ladite flexibilité étant définie ici en termes de résistance à la pliure déterminée par le test à la pliure sur un support cylindrique. Ainsi, le compromis dureté/flexibilité est mauvais ainsi que l'adhérence sur substrats, par exemple dans des applications pour revêtements tels que vernis ou encres. Ceci est essentiellement causé par un taux de réticulation (pouvant s'exprimer par une densité de noeuds de réticulation par unité de poids) trop élevé et par le retrait lié au grand nombre d'insaturations ayant réagi. D'autre part, ces monomères sont basés sur des polyols multifonctionnels spécifiques tels que les diéthers de polyols de plus faible fonctionnalité, par exemple le ditriméthylol propane (DiTMP) ou le dipentaérythritol (DiPE), ces produits étant difficiles d'accès et avec un coût multiple de celui des polyols de départ, par exemple pour le DiTMP par rapport au triméthylol propane (TMP) ou pour le DiPE par rapport au pentaérythritol (PE). On cherche donc une solution pratique, plus simple et moins coûteuse et donc en partant des polyols de départ tels que le TMP ou le PE, ladite solution devant remédier en même temps aux problèmes et inconvénients techniques constatés ci-haut avec les produits existants.

Le recours possible à l'alcoxylation desdits polyols de départ qui permettrait de réduire la densité de réticulation des produits obtenus, entraîne d'autre part une perte de réactivité, ce qui n'est pas acceptable car la réactivité est une des propriétés essentielles exigées, sinon la propriété essentielle de ces monomères. US 5096938 et FR2401946 divulguent des compositions réticulables contenant des polyesters acrylates basés sur la polycondensation de polyols, d'anhydrides et d'acide acrylique présentant de bonnes propriétés de réticulation et d'adhérence.

La solution de la présente invention remédie à ces inconvénients avec des nouveaux produits acrylés ayant des fonctionnalités élevées, sans utiliser des matières premières sophistiquées et coûteuses, tels que des polyéthers ou des structures dendrimères, mais seulement en partant de polyols de base couramment utilisés en chimie et en garantissant une densité de réticulation suffisante et contrôlée pour les produits finaux obtenus, sans être trop élevée et avec un retrait significativement plus faible, avec un compromis entre dureté et flexibilité comme définie ci-haut et une adhérence nettement améliorés. Plus particulièrement, la solution de la présente invention vise des oligomères acrylés multifonctionnels (MFA) de haute fonctionnalité en remplacement des oligomères acrylés MFA courants en apportant une réactivité élevée qui peut être définie comme correspondant à la vitesse minimale de passage sous lampe UV (lampe fusion 120 W/cm$^2$) pour avoir un revêtement sans collant au toucher d'au moins 25 m/min, une dureté plus élevée pouvant se définir comme supérieure ou égale à 150 selon méthode Persoz selon ISO 1522 et de flexibilité inférieure à 32 mm selon ISO 1519 (méthodes telles que définies en partie expérimentale) et ceci sans utilisation de structures aromatiques à base de bisphénol A (BPA) bien connues pour l'obtention de duretés élevées mais au détriment de la flexibilité.

La solution de la présente invention consiste en un produit acrylé qui est un mélange de produits comprenant des oligo(éther-esters) linéaires et ramifiés de structure et de composition contrôlée à partir de polyols courants et de l'acide acrylique et en présence d'au moins un anhydride carboxylique cyclique ou de sa forme polyacide, en proportions en équivalents carboxy minoritaires par rapport à l'acide acrylique avec r1 = CO$_2$H anhydride / CO$_2$H acide acrylique allant de 0,01 à 0,4, de préférence de 0,05 à 0,35 et plus préférentiellement de 0,1 à 0,3, avec une fonctionnalité moyenne élevée et parfaitement contrôlée en acrylates par réactions successives d'estérification et d'éthérification par addition de Michael et d'extension de chaîne contrôlée par formation de diesters à base dudit anhydride carboxylique. Des structures ramifiées de haute fonctionnalité peuvent être formées par l'allongement suffisant via éthérification par addition de Michael et via estérification par l'anhydride carboxylique cyclique ou sa forme polyacide carboxylique et permettent ainsi à la fois une fonctionnalité élevée et une densité de réticulation suffisante sans retrait particulier ni problème d'adhérence ou de compromis dureté/flexibilité et en particulier, une réactivité et une dureté élevées comme déjà définies ci-haut. L'allongement par éthérification (par addition de Michael) est contrôlé par le rapport molaire de l'acide acrylique par rapport aux groupements hydroxyles (OH) dudit polyol, les groupements carboxy de l'acide acrylique et dudit an-

2

hydride ou de sa forme polyacide étant en défaut par rapport auxdits groupements OH et se traduisant par le rapport global r = -CO$_2$H/OH < 1, plus particulièrement inférieur à 0,97.

Parmi les avantages de cette solution par rapport à l'état de l'art antérieur, on peut citer les suivants :

- très bon contrôle d'une structure poly(éther-ester) acrylate (PEEA) de faible viscosité, de faible hydrophilie (la quasi-totalité des groupes hydroxyles sont consommés), de très haute fonctionnalité tout en gardant une densité de doubles liaisons modérée,
- cette structure est contrôlée par la maîtrise des ratios r et r1 définis ci haut, donc par des quantités des réactifs engagées et est peu dépendante de la conversion, ce qui garantit une meilleure reproductibilité des caractéristiques et performances de la production d'un lot à un autre,
- cette structure permet l'obtention de films photoréticulés de haute flexibilité sans perte de dureté et plus particulièrement avec une dureté élevée comme définie ci-haut,
- ces produits présentent une viscosité très inférieure à celle obtenue par simple polyestérification avec ajout d'un diacide comme réactant principal avec ledit polyol ou par une polyéthérification par simple déshydratation,
- un autre avantage particulier et important est leur synthèse très simple et très pratique qui ne nécessite qu'une seule étape, en partant d'un mélange réactif d'un polyol ou un mélange de polyols courants et en présence d'un anhydride carboxylique ou de sa forme polyacide en proportions en équivalents carboxy minoritaires par rapport à l'acide acrylique, avec globalement les groupements carboxy de l'acide acrylique et dudit anhydride carboxylique ou de sa forme polyacide étant en défaut stoechiométrique par rapport aux groupements OH dudit polyol comme seuls réactants et avec une catalyse acide et un reflux hétéro-azéotropique pour extraire l'eau d'estérification et sans aucun besoin de séparation/purification du produit final. Contrairement aux produits courants connus ou décrits, par exemple dans JP 2010024380, le produit n'est pas lavé mais juste neutralisé, d'où une meilleure empreinte carbone par réduction des effluents avec le rendement limité par la seule perte de l'eau d'estérification,
- l'indice d'hydroxyle final est très faible, d'où une faible hydrophilie par rapport à la tolérance à l'eau ou une grande hydrophobie, ce qui minimise l'impact sur l'environnement,
- plus particulièrement, une très grande réactivité correspondant à une vitesse minimale de passage sous lampe UV d'au moins 25 m/min et une dureté élevée, c'est-à-dire d'au moins 150 en Persoz selon ISO 1522 et une flexibilité n'atteignant pas 32 mm selon ISO 1519, sans aucune utilisation de structures de type bisphénol A, couramment utilisées dans l'état de la technique pour atteindre une telle performance en dureté.

Parmi les autres avantages de la solution selon la présente invention, on peut citer en particulier le fait que le produit selon l'invention est un mélange de produits acrylés de structure et de composition bien contrôlée et reproductible, obtenu en une seule étape, utilisable directement comme tel pour l'application finale, sans nécessiter d'opérations coûteuses de séparation de sous-produits, avec un procédé de préparation simple et pratique à mettre en oeuvre. Un autre avantage particulier de ce produit final est le fait qu'il présente une distribution moléculaire avec présence contrôlée du monomère acrylé de départ qui joue le rôle de diluant réactif pour la composition d'application. Par conséquent, le produit final ne nécessite pas, en général, l'addition de diluant réactif supplémentaire pour ajuster sa viscosité. Par contre, il est possible d'utiliser un tel diluant réactif supplémentaire pour les masses moléculaires moyennes du produit final les plus élevées, ceci en fonction de l'application finale et de la viscosité d'application requise. Un avantage particulier desdits produits de l'invention est leur faible retrait volumique malgré leur fonctionnalité élevée en acrylates. Ceci est spécifique à la présente invention.

L'invention concerne d'abord un produit acrylé, en particulier oligomère acrylé multifonctionnel, lequel est le produit de réaction d'un polyol ou d'un mélange spécifique de polyols, de l'acide acrylique en présence d'au moins un anhydride carboxylique ou de sa forme polyacide carboxylique en proportions en équivalents carboxy minoritaires, par rapport à l'acide acrylique et avec obtention d'un mélange de monomères et d'oligomères multifonctionnels acrylés, par réaction à la fois d'estérification des hydroxyles libres par l'acide acrylique, d'éthérification via addition de Michael sur la double liaison acrylate et d'acide acrylique desdits groupements hydroxyles globalement en excès par rapport aux groupements carboxy et hydroxyles portés par les esters partiels acrylates et extension limitée de chaîne par condensation dudit anhydride carboxylique ou polyacide avec lesdits hydroxyles.

Ledit produit de l'invention est également défini de manière indépendante et alternative comme le produit susceptible d'être obtenu par un procédé spécifique défini par des conditions spécifiques de procédé.

Un autre objet de l'invention concerne un procédé d'obtention dudit produit acrylé en tant que mélange de produits acrylés qui sont des oligomères acrylés multifonctionnels.

Un autre objet couvert par la présente invention concerne une composition réticulable comprenant au moins un produit acrylé tel que défini selon la présente invention.

Ensuite, l'invention couvre également l'utilisation dudit produit acrylé selon l'invention comme liant acrylé multifonctionnel dans des compositions réticulables, en particulier avec une haute densité de réticulation et un faible retrait, plus particulièrement dans des compositions de revêtements pigmentés ou non pigmentés, en particulier des peintures, vernis, encres, adhésifs ou dans des compositions de moulage, d'étanchéité ou de composite ou de scellement chimique ou d'impression en 3D ou d'objets en 3D réalisés couche par couche.

Finalement, l'invention couvre des produits finis obtenus par l'utilisation d'au moins un produit acrylé selon l'invention ou par réticulation d'une composition réticulable de l'invention comprenant ledit produit acrylé, lesdits produits étant sélectionnés parmi : des revêtements pigmentés ou non pigmentés, en particulier des peintures, vernis, encres, adhésifs ou parmi des compositions de moulage, d'étanchéité ou de composite ou de scellement chimique ou d'impression en 3D ou d'objets en 3D réalisés couche par couche.

Donc, le premier objet de la présente invention concerne un produit acrylé multifonctionnel, en particulier oligomère acrylé multifonctionnel, caractérisé en ce qu'il a une fonctionnalité moyenne en nombre f supérieure à 2,1, de préférence d'au moins 2,5 et plus préférentiellement de 2,75 à 20 groupements acrylates et encore plus préférentiellement de 3 à 14 groupements acrylates par mole dudit produit et en particulier avec une densité desdits groupements acrylates DA allant de 2 à 12 mmoles par g dudit produit, ledit produit étant le produit de réaction par estérification et par éthérification par réaction d'addition de Michael, entre :

a) un polyol $R(OH)_m$ ou un mélange de polyols $R(OH)_m$, de fonctionnalité m d'au moins 3, de préférence de 3 à 6, plus préférentiellement de 4 à 6 pour un seul polyol présent et une fonctionnalité OH moyenne en nombre supérieure à 2,1, de préférence supérieure à 2,3, plus préférentiellement d'au moins 2,5 et jusqu'à 6, pour un mélange desdits polyols et

b) l'acide acrylique représenté par $R_1OH$,

ladite réaction entre a) et b) ayant lieu en présence de c) au moins un anhydride carboxylique cyclique ou de sa forme polyacide carboxylique $R_2(CO_2H)z$, de fonctionnalité z en groupements carboxy ($-CO_2H$) d'au moins 2 et allant jusqu'à 4, de préférence de 2 à 3, plus préférentiellement 2, avec :

- le rapport $r_1$ en nombre de groupements carboxy dudit anhydride c) par rapport à ceux de b) acide acrylique, $r_1 = (CO_2H)_c / (CO_2H)_b$ allant de 0,01 à 0,4, de préférence de 0,05 à 0,35 et plus préférentiellement de 0,1 à 0,3,
- les groupements carboxy étant globalement en défaut par rapport aux groupements hydroxy dudit polyol a), avec $r = CO_2H/OH < 1$, en particulier inférieur ou égal à 0,97

ledit produit acrylé comprenant dans sa composition à la fois :

- des unités A) d'oligoéther-ester acrylate issues de la réaction de a) et de b), formées par une réaction d'addition de Michael :

  - des groupements OH dudit polyol a) ou
  - de groupements OH d'acrylates partiels hydroxylés formés sur l'insaturation de l'acide acrylique b) ou sur l'insaturation d'un des acrylates formés par estérification par b) et estérification simultanée par b) dudit polyol a) et desdits acrylates partiels hydroxylés ou (estérification simultanée) par les groupements carboxy de l'adduit carboxylé de Michael formé entre a) et b) et

- des unités B) oligoesters acrylates issues de c) par réaction d'estérification par ledit anhydride ou par sa forme polyacide c) dudit polyol a) ou desdits acrylates partiels hydroxylés ou des éther-ester acrylates hydroxylés formés

ledit produit acrylé étant un mélange de produits acrylés comprenant au moins un produit acrylé p1 associant chimiquement dans sa structure moléculaire les deux types d'unités A) et B) comme définies ci-dessus.

Plus particulièrement, le produit acrylé selon l'invention a une composition globale qui peut être représentée par la formule moyenne générale (I) suivante :

$$R_1O\text{-}[[A]_a\text{-}[B]_b]_n\text{-}R(OR_1)_{m-1} \qquad (I)$$

avec a et b représentant la fraction molaire moyenne de chaque unité A) et B) par unité moyenne globale dudit produit et avec a + b = 1 et a/b allant de 0,15 à 22, de préférence de 0,5 à 10, plus préférentiellement de 1 à 5, n étant le nombre d'unités (motifs) globales répétitives avec n moyen par mole dudit produit $n_{moy}$ allant de 0,2 à 10, de préférence de 0,35 à 8, plus préférentiellement de 0,35 à 6 et encore plus préférentiellement de 0,4 à 2,5.

Il est évident, comme le montrent les formules plus détaillées, que les unités A) et B) telles que définies ci-avant portent des groupements latéraux acrylates.

Selon une option particulière, ledit produit acrylé de l'invention comprend ledit produit p1 et ledit produit p1 a une structure moléculaire définie selon la formule générale (II) suivante :

$$(R_1O)_{m-1}\text{-}R\text{-}[\text{-}[O\text{-}CH_2CH_2\text{-}CO_2\text{-}R(OR_1)_{m-2}]_a[O_2C\text{-}R2(CO_2X)_{z-2}\text{-}CO2\text{-}R(OR_1)_{m-2}]_b]_n\text{-}OR1 \qquad (II)$$

et avec présence d'au moins 4 produits de n différent, correspondant à n = 0 et n = 1, n = 2 et n = 3
avec :

- $R_1$ étant le radical acryloyle, R étant le radical résiduel dudit polyol $R(OH)_m$ ou représentant un radical moyen d'un mélange de polyols,
- $R_2$ étant le résidu de valence z dudit anhydride ou sa forme polyacide carboxylique et X étant $-R(OR_1)_{m-1}$ où X pouvant être essentiellement, c'est-à-dire à plus de 95% $-R(OR_1)_{m-1}$ et le reste (moins de 5%) de X étant H, en particulier avec un indice d'acide ne dépassant pas 15 mg KOH/g, plus particulièrement ne dépassant pas 10 mg KOH/g
- n étant le nombre d'unités répétitives et
- a et b étant les fractions molaires respectives des unités particulières dans l'unité répétitive globale avec le rapport a/b allant de 0,15 à 22, de préférence de 0,5 à 10, plus préférentiellement de 1 à 5.

Selon une option encore plus particulière, ledit produit de l'invention comprend en plus dudit produit p1, un produit p2 oligoéther-ester acrylate à base d'unités A) de formule générale (III) suivante :

$$(R_1O)\text{-}[\text{-}R[OR_1]_{m-2}\text{-}O\text{-}(C=O)\text{-}CH_2\text{-}CH_2\text{-}O\text{-}]_n\text{-}R\text{-}(OR_1)_{m-1} \qquad (III)$$

avec présence d'au moins 4 produits de n différent et correspondant à n = 0 et n = 1, n = 2 et n = 3 et n étant le nombre d'unités répétitives.

Selon une option plus spécifique, il comprend en plus dudit produit p1, un produit p3 oligoester acrylate de formule générale (IV) suivante :

$$(R_1O)\text{-}[\text{-}R[OR_1]_{m-2}\text{-}O\text{-}(C=O)\text{-}R2(C=O)O\text{-}]_n\text{-}R\text{-}(OR_1)_{m-1} \qquad (IV)$$

avec présence d'au moins 4 produits de n différent et correspondant à n = 0 et n = 1, n = 2 et n = 3 et n étant le nombre d'unités répétitives.

Selon une option encore plus spécifique et préférée, ledit produit comprend un produit p2 tel que défini selon la formule (III) décrite ci-haut, ledit produit p1 est tel que défini selon la formule (II) telle que définie ci-haut et que les 3 produits p1, p2 et p3 ainsi définis comprennent chacun au moins un cinquième produit correspondant à n = 4 et en option un produit supplémentaire correspondant à n = 5.

Plus particulièrement, ledit produit p1 est de formule générale suivante (V) :

$$(R_1O)\text{-}[\text{-}R[OR_1]_{m-2}\text{-}O_2C\text{-}CH_2\text{-}CH_2\text{-}O\text{-}]_n\text{-}R\text{-}(OR_1)_m\text{-}2\text{-}O_2C\text{-}R_2(CO2\text{-}X_1)_{z-2}\text{-}CO_2\text{-}X_2 \qquad (V)$$

avec $R_2$ étant le radical de valence z correspondant audit anhydride carboxylique ou audit acide polycarboxylique et $X_1$ et $X_2$ pouvant être identiques ou différents et choisis parmi :

- $-R(OR_1)_{m-1}$ ou
- $-R(OR_1)_{m-2}\text{-}[O\text{-}CH_2\text{-}CH_2\text{-}CO_2\text{-}R(OR_1)_{m-2}]_n\text{-}(OR_1)$ ou
- en partie H, en particulier avec un indice d'acide correspondant ne dépassant pas 20, en particulier ne dépassant pas 15 mg KOH/g ou
- en partie résidu d'un agent bloquant réactif monofonctionnel, en particulier réactif avec le groupement carboxy résiduel en particulier par estérification desdits carboxy résiduels d'indice d'acide résiduel ne dépassant pas 20, plus particulièrement ne dépassant pas 15 mg KOH/g.

Concernant le cas où $X_1$ et/ou $X_2$ sont en partie des groupements carboxy résiduels avec l'indice d'acide correspondant ne dépassant pas 20 mg KOH/g, en particulier ne dépassant pas 15, plus particulièrement si la réaction d'estérification est incomplète avec ledit anhydride ou ledit polyacide, ledit agent boquant monofonctionnel peut être un monoépoxyde saturé ou insaturé. Comme exemple de monoépoxyde insaturé, on peut citer le glycidyl méthacrylate et comme monoépoxyde saturé les dérivés éthers ou esters de glycidyle respectivement avec un alcool ou un acide carboxylique.

Plus particulièrement, la distribution moléculaire globale en n dudit produit est telle qu'elle représente au moins 80% en poids pour n allant de 0 à 4 et pas plus de 20% en poids de ladite distribution pour n étant supérieur à 4, de préférence avec une masse moyenne en nombre Mn dudit produit mesurée par GPC dans le THF et exprimée en équivalents polystyrène allant de 500 à 10000 et plus préférentiellement de 600 à 6000. Ladite Mn dudit produit est calculée à partir de n moyen tel que défini plus haut le poids moyen moléculaire de l'unité répétitive globale considérée.

Selon une option particulière dudit produit selon l'invention, ledit polyol a) a une fonctionnalité m pour un polyol a) seul, d'au moins 3 et ledit produit comprend des produits p2 oligoéther-ester acrylates linéaires selon la formule générale (III) et en plus au moins un produit oligoéther-ester acrylate de structure ramifiée (ou à chaîne ramifiée). Le terme de structure « ramifiée » signifie pour le produit considéré qu'il y a au moins un chaînon latéral de même nature et attaché par liaison covalente sur la chaîne principale dudit produit.

Ledit produit acrylé alternativement peut être défini comme le produit susceptible d'être obtenu par réactions simultanées ou successives et alternées entre a) un polyol $R(OH)_m$ ou mélange de polyols $R(OH)_m$, de fonctionnalité m d'au moins 3, de préférence de 3 à 6, plus préférentiellement de 4 à 6, pour un polyol a) seul ou de fonctionnalité moyenne en nombre (sur m) supérieure à 2,1, de préférence supérieure à 2,3, plus préférentiellement d'au moins 2,5 et jusqu'à 6 pour un mélange de polyols $R(OH)_m$ et b) l'acide acrylique ($R_1OH$) en défaut par rapport à a) et en présence de c) au moins un anhydride carboxylique cyclique ou de sa forme polyacide de fonctionnalité z en groupements carboxy allant de 2 à 4, de préférence de 2 à 3, plus préférentiellement de 2, avec un rapport global $r = CO_2H/OH$, inférieur à 1, en particulier supérieur à $[(m-1)*(r_1+1)]/[m*((r_1/2)+1)]$ et allant jusqu'à 0,97, de préférence supérieur à $1,05*[(m-1)*(r_1+1)]/[m*((r_1/2)+1)]$ et allant jusqu'à 0,97 et plus préférentiellement supérieur à $1,10*[(m-1)*(r_1+1)]/[m*((r_1/2)+1)]$ et allant jusqu'à 0,97, avec $r_1$ étant le rapport des groupements carboxy de c) sur les groupements carboxy de b) acide acrylique $r_1 = (carboxy)_c/(carboxy)_b$, plus particulièrement avec z = 2.

Ledit polyol a), seul ou en mélange, peut être sélectionné parmi des monomères polyols et/ou des oligomères polyols avec Mn pour oligomères polyols ne dépassant pas 700, de préférence ne dépassant pas 600. Cette valeur Mn pour lesdits oligomères polyols peut être calculée à partir de l'indice OH et de la fonctionnalité.

Pour un polyol a) qui est un monomère polyol, il peut être sélectionné parmi : diéthylène glycol, triéthylène glycol, tetraéthylène glycol, propylène glycol, dipropylène glycol, tripropylène glycol, tetrapropylène glycol, butane diol, néopentyl glycol, hexane diol, isosorbide, glycérol, triméthylol propane, pentaérythritol, ditriméthylol propane, érythritol, xylitol, dipentaérythritol, sorbitol, y compris les dérivés alcoxylés des polyols cités.

Le polyol a) peut être un oligomère polyol choisi parmi : polyéther polyols, polyester polyols, oligomères acryliques hydroxylés, en option alcoxylés et en particulier, il est présent en mélange avec au moins un autre polyol a), de préférence un polyol monomère tel que défini ci-avant.

De préférence, quand ledit polyol a) est alcoxylé, il y a de 1 à 4 unités alcoxy. Un oligomère polyol convenable peut être sélectionné parmi : polyéther polyols, polyester polyols, oligomères acryliques hydroxylés. Les oligomères acryliques hydroxylés peuvent être par exemple des oligomères copolymères à base d'hydroxy alkyl (méth)acrylates, avec le taux dudit hydroxy alkyl (méth)acrylate fixant la fonctionnalité dudit oligomère.

Ledit produit de l'invention peut être obtenu comme déjà décrit ci-haut à partir d'un mélange de polyols a) comme définis ci-haut.

Selon une option particulière et préférée du produit de l'invention en plus dudit polyol a) de fonctionnalité d'au moins 3, est présent un deuxième polyol différent du premier de fonctionnalité d'au moins 2, de préférence ce deuxième polyol étant un oligoester diol, plus préférentiellement comprenant comme composant dans son unité répétitive ledit anhydride c) ou sa forme polyacide. Cette option permet ainsi d'ajuster la fonctionnalité moyenne f et la compatibilité entre les composants du mélange des produits acrylés compris dans le produit acrylé selon l'invention, en particulier les produits tels que définis ci-haut selon p1, p2 ou p3.

Le terme anhydride carboxylique, tel qu'utilisé dans la présente invention, signifie un anhydride carboxylique cyclique qui peut être aliphatique, cycloaliphatique ou aromatique. Sa forme polyacide carboxylique correspondant audit anhydride, tel qu'utilisé dans la présente invention, signifie le polyacide carboxylique correspondant à l'ouverture (hydrolyse) dudit cycle anhydride carboxylique. Ce terme polyacide carboxylique comprend dans sa signification tous les isomères de position dudit polyacide carboxylique. Dans le cas où la fonctionnalité z dudit anhydride est un nombre impair, cela signifie que le nombre de groupements anhydrides par molécule est égal à (z-1)/2 et qu'il porte en plus un groupement carboxy non associé par un cycle anhydride.

En particulier, ledit anhydride polycarboxylique c) ou sa forme polyacide ou isomère de ce dernier peut être choisi parmi les anhydrides polycarboxyliques ou polyacides carboxyliques aromatiques ou cycloaliphatiques ou aliphatiques, avec les structures cycloaliphatiques ou aliphatiques pouvant être insaturées.

Ledit anhydride polycarboxylique ou polyacide ou ledit isomère de ce dernier peut être aromatique et en particulier sélectionné parmi : l'anhydride phtalique (o-), le diacide iso ou téréphtalique, anhydride ou diacide naphténique, anhydride ou polyacide trimellitique, anhydride pyromellitique ou tetra-acide pyromellitique, de préférence ledit anhydride ou polyacide ayant une fonctionnalité de 2.

Ledit anhydride ou sa forme polyacide peut être aussi sélectionné parmi les anhydrides et leur forme diacides cycloaliphatiques, en particulier parmi l'anhydride et diacide tétrahydrophtalique, l'anhydride et diacide dihydrophtalique, l'anhydride et diacide nadique (bicyclo (2,2,1) hept-5-ène 2, 3 dicarboxylique) ou l'anhydride et diacide cyclohexane dicarboxylique.

Selon une option particulière, ledit anhydride ou acide peut être aliphatique et en particulier choisi parmi : anhydride et acide maléique, diacide fumarique, anhydride et diacide itaconique, anhydride et diacide succinique.

Selon une autre option particulière, ledit anhydride ou acide est choisi parmi : le phtalique (o-), le diacide iso ou téréphtalique, l'anhydride et diacide tetrahydrophtalique, l'anhydride et diacide dihydrophtalique, l'anhydride et diacide nadique, l'anhydride et diacide maléique, le diacide fumarique, l'anhydride et diacide itaconique, l'anhydride et diacide succinique.

Ledit anhydride ou sa forme polyacide peut être un mélange d'au moins deux anhydrides et/ou polyacides c).

La longueur de chaîne dudit produit acrylé selon l'invention, y compris selon les formules (I), (II), (III), (IV) et (V), est caractérisée par l'indice n, lequel correspond au nombre de motifs (ou unités) A) ou B) ou motifs globaux (ou unités) moyens, le motif moyen étant une moyenne sur les unités de type A) et B) compte tenu de leur proportions molaires sur un motif global moyen. En effet, ledit produit comprend des motifs éther-ester enchaînés (oligoéther-esters) de type A, par réactions successives d'addition de Michael, d'un OH dudit polyol sur l'acide acrylique, suivies de l'estérification d'un OH résiduel (parmi m-1) dudit polyol par une autre molécule d'acide acrylique dont l'insaturation peut à nouveau faire l'objet d'une autre addition de Michael par un OH d'une autre molécule dudit polyol et des motifs (ou unités) B) esters enchaînés (oligoesters) par réaction simultanée dudit anhydride ou de sa forme polyacide c) avec les dérivés hydroxylés, y compris polyol a), acrylates partiels hydroxylés et éther-ester acrylates hydroxylés, avec extension de chaîne supplémentaire ou séparée.

En particulier sur la base de la formule globale (I), les indices moyens n*a et n*b pour chaque type de motif (unité) A) et B) peuvent être estimés, en particulier pour z = 2 pour une conversion totale à partir du rapport en équivalents r = $CO_2H$/OH, le ratio r1 = COOH anhydride / COOH acide et la fonctionnalité m dudit polyol par la relation suivante :

$$n_{MOY}\text{*}a = [\ m\text{*}(1\text{-}r)\text{*}(r_1\text{+}1)\ ]\ /\ [\ m\text{*}r\text{*}((r_1/2)\text{+}1)\text{+}(1\text{-}m)\text{*}(r_1\text{+}1)\ ]$$

$$n_{MOY}\text{*}b = [\ m\text{*}r\text{*}r_1\ ]\ /\ [\ m\text{*}r\text{*}((r_1/2)\text{+}1)\text{+}(1\text{-}m)\text{*}(r_1\text{+}1)\ ]$$

Il est rappelé que $n_{moy}\text{*}a + n_{moy}\text{*}b = n$ moyen ($n_{moy}$), puisque a + b = 1.

Une fonctionnalité moyenne en groupements acrylates f par produit acrylé peut être estimée (calculée) à partir de n moyen $n_{moy}$ décrit ci-haut, de la fonctionnalité m dudit polyol et de la formule (I). Ainsi, f est définie, en particulier pour z = 2, selon formule suivante :

$$f = (m\text{-}2)\text{*}n_{moy} + m$$

A noter que dans le cas d'un mélange de 2 polyols de fonctionnalités m1 et m2 à des taux molaires respectifs x1 et x2 (x1 + x2 = 1) dans ledit mélange, dans ce cas, la fonctionnalité m à utiliser est la moyenne en nombre (molaire) des deux polyols selon la relation suivante :

$$m\ moyen = x1\text{*}m1 + x2\text{*}m2$$

Dans le cas d'un mélange de plusieurs polyols d'indice i de fonctionnalité $m_i$ et de taux molaires $x_i$ ($\sum_i x_i = 1$), la fonctionnalité moyenne m sera égale à $m = \sum_i x_i \text{*} m_i$.

De préférence, n moyen $n_{moy}$ varie de 0,2 à 10, de préférence de 0,35 à 8, plus préférentiellement de 0,35 à 6 et encore plus préférentiellement de 0,4 à 2,5.

Ledit produit acrylé de l'invention peut comprendre des acrylates de structure linéaire et/ou ramifiée. Par définition, il ne peut comporter de structure réticulée laquelle est ainsi exclue. Structure linéaire signifie ici une chaîne linéaire avec possibilité de porter des groupements acrylates latéraux et une chaîne ramifiée dérive d'une telle chaîne essentiellement par l'addition de Michael sur lesdits acrylates latéraux avec formation de chaînons éther-ester acrylates latéraux.

Un deuxième objet de l'invention concerne un procédé de préparation dudit produit acrylé tel que défi ni ci-haut selon l'invention.

Ledit procédé de préparation d'un produit tel que défini selon l'invention comprend les étapes suivantes :

i) le mélange dans un réacteur dudit polyol a) de l'acide acrylique b), dudit anhydride ou dudit polyacide c) et dans des proportions telles que le rapport molaire global r = $CO_2H/OH$ soit inférieur à 1, en particulier supérieur à $[(m-1)*(r_1+1)]/[m*((r_1/2)+1)]$ et allant jusqu'à 0,97, de préférence supérieur à $1,05*[(m-1)*(r_1+1)]/[m*((r_1/2)+1)]$ et allant jusqu'à 0,97 et plus préférentiellement supérieur à $1,10*[(m-1)*(r_1+1)]/[m*((r_1/2)+1)]$ et allant jusqu'à 0,97, avec $r_1$ = $CO2H_c/CO2H_b$ étant dans la plage de 0,01 à 0,4, de préférence de 0,05 à 0,35 et plus préférentiellement de 0,1 à 0,3, plus particulièrement pour z = 2 et
en présence d'un catalyseur acide d'estérification et d'un solvant formant un azéotrope avec l'eau pour former le mélange réactionnel, suivi de
ii) la mise sous reflux dudit mélange réactionnel, avec réactions simultanées ou successives et alternées d'estérification, par réaction de l'acide acrylique b) et /ou dudit anhydride ou polyacide c) avec un hydroxy dudit polyol a) avec formation d'esters acrylates hydroxylés et d'éthérification par réaction d'addition de Michael d'un hydroxy dudit polyol ou dudit acrylate hydroxylé formé sur un groupement acrylate et/ou l'acide acrylique b) et d'estérification des groupements hydroxy dudit polyol et desdits acrylates hydroxylés par ledit anhydride ou diacide et élimination progressive de l'eau d'estérification, avec
iii) poursuite de la réaction jusqu'à consommation complète des fonctions OH ($I_{OH}$<20 mg KOH/g) par réactions d'addition de Michael et d'estérification avec ledit acide acrylique b) et ledit anhydride ou diacide c),
iv) neutralisation dudit catalyseur acide avant récupération du produit final, par élimination dudit solvant, sans aucune autre étape spécifique de purification requise.

Un autre objet de la présente invention concerne une composition réticulable, laquelle comprend au moins un produit de l'invention tel que décrit ci-haut ou obtenu par le procédé décrit ci-avant.

Dans le cas particulier où la Mn calculée dudit produit acrylé selon l'invention est supérieure à 1000, de préférence supérieure à 1500, ladite composition peut comprendre en plus dudit produit, au moins un diluant réactif, sélectionné parmi les monomères acryliques de préférence multifonctionnels. Le rôle essentiel de ce diluant, s'il y a besoin, est d'ajuster la viscosité en fonction de l'application finale.

Plus particulièrement, ladite composition est une composition réticulable, de préférence par rayonnement, en particulier par rayonnement UV en présence d'un système photoamorceur ou par faisceau d'électrons (EB) en l'absence de système photoamorceur et/ou par un système d'amorçage radicalaire thermique, en particulier par système d'amorçage peroxyde (P-cure) et/ou par addition de Michael (M-cure) ou par un système mixte, en particulier par réticulation duale (dual cure), plus particulièrement avec présence d'au moins deux des systèmes de réticulation précités.

L'invention couvre également l'utilisation dudit produit tel que décrit ci-haut ou obtenu par un procédé tel que défini selon l'invention dans des compositions réticulables présentant un faible taux de retrait, de préférence dans des compositions réticulables de revêtements. Plus particulièrement, une telle utilisation s'applique à des compositions de revêtements pigmentés ou non pigmentés, de préférence parmi peinture, vernis, encre, adhésif ou une composition de moulage ou une composition d'étanchéité ou une composition de composite ou une composition de scellement chimique ou une composition pour impression 3D ou une composition pour objets en 3D couche par couche.

Finalement, l'invention concerne aussi le produit fini réticulé en particulier choisi parmi les revêtements pigmentés ou non pigmentés réticulés, de préférence parmi les peintures, vernis, encres, adhésifs ou produit fini choisi parmi les pièces moulées, les joints d'étanchéité, les composites, les scellements chimiques, impressions 3D ou objets en 3D couche par couche, lequel produit fini résulte de l'utilisation d'au moins un produit tel que défini ci-haut ou obtenu par un procédé tel que défini ci-haut selon l'invention ou de la réticulation d'une composition réticulable comme définie ci-haut selon l'invention.

Les exemples qui suivent sont présentés au titre d'illustration de l'invention et de ses performances et ne limitent nullement la couverture de l'invention.

<u>EXEMPLES</u>

1) <u>Matières premières utilisées (voir tableau 1)</u>

Tableau 1 : matières premières utilisées

| Nom commercial (REF) | Nom chimique | Nom abrégé | Fournisseur | Fonction selon l'invention | Fonctionnalité |
|---|---|---|---|---|---|
| TMP (Hydro) flakes | Tri méthylol propane | TMP | BASF | Polyol a) | 3 |
| TEG | Tri éthylène glycol | TEG | SABIC | Polyol a) | 2 |
| Anhydride Phtalique | Anhydride phtalique | AnPt | ATMOSA | Anhydride Carboxylique c) | 2 |
| Acide acrylique glacial | Acide acrylique | AA | Arkema | Acide acrylique b) | 1 |
| Toluène | Toluène | Tol | TOTAL | Solvant | |
| MSA E-pure | Acide méthane sulfonique | AMS | Arkema | Catalyseur | |
| Hydroquinone Extra pure | Hydroquinone | HQ | Rhodia | Inhibiteur | |
| TIB KAT® 256 | Oxyde de mono butyl Etain | OMBE | TIB Chemicals | Catalyseur | |
| Darocur® 1173 | 2-Hydroxy-2-méthyl-phényl-propane-1-one | PI-1 | BASF | Photoamorceur | |
| Dipropylamine | Dipropylamine | DPA | BASF | Agent neutralisant vs catalyseur | |
| Grilonit® V51-31 | Tri glycidyle éther de tri méthylol propane | TGETMP | EMS | Agent neutralisant vs b) pour réduire b) résiduel | |
| Triphenyl phosphine | TriPhényle Phosphine | TPP | Rhodia | Catalyseur COOH vs Epoxy | |

2) Préparation des produits selon l'invention

2.1) Mode opératoire pour exemples selon l'invention (exemples 1 à 2)

Les rapports r et $r_1$ cités ci-dessous dans les exemples correspondent :

- r, au rapport global d'équivalents -$CO_2H$/-OH,
- $r_1$, au rapport d'équivalents -$CO_2H$ provenant de l'anhydride ou du diacide c) / - COOH provenant de l'acide acrylique b) ou $r_1 = (CO_2H)_c/(CO_2H)_b$.

Exemple 1

**Préparation d'un polyester hydroxylé (diol) A-1 utilisé comme polyol a)**

Dans un réacteur de 1 litre équipé d'un agitateur à ancre et surmonté d'un florentin avec son réfrigérant (dispositif permettant le soutirage continu de l'eau d'estérification), d'une entrée d'azote (pour l'inertage) et d'une sonde thermométrique, on introduit : 669,1 g de triéthylène glycol (4,461 mole), 329,9 g d'anhydride phtalique (2,229 mole) et 1,0 g d'oxyde de monobutyle étain (0,005 mole).

Le mélange réactionnel est porté progressivement à deux paliers de température 100 et 150°C pendant 5 heures, puis à 210-220°C pendant 7 heures, jusqu'à un indice d'acide inférieur à 5 mg KOH/g au moment de l'arrêt de l'estérification effectué par refroidissement du milieu réactionnel, après avoir distillé 4,0 ml d'eau.

On obtient un produit Polyester hydroxylé A-1 ayant les caractéristiques suivantes :

Aspect : limpide

Acidité résiduelle ou indice d'acide du produit : 3,0 mg KOH/g

Indice OH du produit : 260 mg KOH/g

Mn calculée à partir de l'indice OH et une fonctionnalité de 2, donnant Mn = 430 g/mole.

## Préparation d'un Poly Phtalo-éther-ester Acrylate B-1 selon l'invention

On utilise comme polyol un mélange de polyols à base du polyol (diol) hydroxylé A-1 de l'exemple 1 et de TMP et avec r = 0,926 et $r_1$ = 0,191.

Dans un réacteur de 1 litre équipé d'un agitateur à ancre et surmonté d'un florentin avec son réfrigérant (dispositif permettant le soutirage continu de l'eau d'estérification sous reflux de solvant), d'une entrée d'air (barbotage d'air) et d'une sonde thermométrique, on introduit : 264,8 g d'acide acrylique (AA) (3,678 mole), 212,3 g de Polyester diol A-1 de l'exemple 1 (0,494 mole), 167,1 g de Triméthylol propane (TMP) (1,247 mole), 51,9 g d'anhydride phtalique (0,351 mole), 250,7 g de toluène, 10,3 g d'acide méthane sulfonique en solution aqueuse à 70% (AMS-aq) (0,075 mole), 3,7 g d'hydroquinone (HQ) (0,003 mole).

Le mélange réactionnel est mis sous reflux pendant 10 heures, passant ainsi d'une température de 105°C (début d'ébullition) à 115°C, jusqu'à un indice d'acide faible < 15 mg KOH/g au moment de l'arrêt de l'estérification effectué par refroidissement du milieu réactionnel, après avoir distillé 75 ml d'eau.

Cette phase organique est neutralisée à 50°C par l'ajout de 10,6 g de dipropylamine (0,105 mole) et sous agitation pendant 1 h avant distillation sous vide à 80-95°C et 200-100 millibars jusqu'à élimination complète du solvant avec un toluène résiduel < 0,1%. On ajoute alors 25,5 g de triglycidyle éther de Triméthylol propane (0,084 mole) et 3,1 g de triphényle phosphine (0,012 mole) puis on élève progressivement la température jusqu'à 13°C que l'on maintient jusqu'à un indice d'acide résiduel < 10 mg KOH/g.

On obtient un produit polyphtalo(éther-ester)acrylate B-1 ayant les caractéristiques suivantes :

| | |
|---|---|
| Aspect : | limpide |
| Turbidité : | 9% |
| Viscosité à 25°C : | 8 Pa.s |
| Acidité résiduelle ou indice d'acide du produit : | 7 mg KOH/g |
| Indice OH du produit : | < 20 mg KOH/g. |

Les caractéristiques moléculaires de B-1 sont présentées au tableau 2 ci-dessous.

On réalise, par simple mélange à froid, une formulation F-1 selon la composition centésimale suivante :

B-1 : 96%

PI-1 : 4%

## Caractéristiques de la formulation F-1

| | |
|---|---|
| Réactivité : | 30 m/min |
| Dureté Persoz : | 180 s |
| Flexibilité : | 20 mm |
| Résistance à l'acétone : | > 300 s |

Exemple 2

## Préparation d'un polyphtalo-éther-ester acrylate B-2 selon l'invention

On utilise comme polyol a) leTMP avec r = 0,836 et $r_1$ = 0,235.

Dans un réacteur de 1 litre équipé d'un agitateur à ancre et surmonté d'un florentin avec son réfrigérant (dispositif permettant le soutirage continu de l'eau d'estérification sous reflux de solvant), d'une entrée d'air (barbotage d'air) et d'une sonde thermométrique, on introduit: 324,8 g d'acide acrylique (AA) (4,511 mole), 297,6 g de triméthylolpropane (TMP) (2,221 mole), 78,4 g d'anhydride phtalique (0,529 mole), 244,3 g de toluène, 13,7 g d'acide méthane sulfonique en solution aqueuse à 70% (AMS-aq) (0,100 mole), 1,5 g d'hydroquinone (HQ) (0,001 mole).

Le mélange réactionnel est mis sous reflux pendant 18 heures, passant ainsi d'une température de 105°C (début d'ébullition) à 115°C, jusqu'à un indice d'acide faible < 15 mg KOH/g au moment de l'arrêt de l'estérification effectué par refroidissement du milieu réactionnel, après avoir distillé 95 ml d'eau.

Cette phase organique est neutralisée à 50°C de 14,2 g de dipropylamine (0,140 mole) et sous agitation pendant 1 h avant distillation sous vide à 80-95°C et 200-100 millibars jusqu'à élimination complète du solvant (avec toluène résiduel < 0,1%). On ajoute alors 22,3 g de triglycidyle éther de triméthylol propane (0,074 mole) et 3,3 g de triphényle phosphine (0,013 mole) puis on élève progressivement la température jusqu'à 125°C que l'on maintient jusqu'à un indice d'acide résiduel < 10 mg KOH/g.

On obtient un produit polyphtalo(éther-ester)acrylate B-2 ayant les caractéristiques suivantes :

| | |
|---|---|
| Aspect : | limpide |
| Turbidité : | 10% |
| Viscosité à 50°C : | 20 Pa.s |
| Acidité résiduelle ou indice d'acide du produit : | 8 mg KOH/g |
| Indice OH du produit : | < 20 mg KOH/g. |

Les caractéristiques moléculaires de B-2 sont présentées au tableau 2 ci-dessous.

On réalise, par simple mélange à froid, une formulation F-2 selon la composition centésimale suivante :

B-2 : 96%

PI-1 : 4%

**Caractéristiques de la formulation F-2**

| | |
|---|---|
| Réactivité : | 40 m/min |
| Dureté Persoz : | 290 s |
| Flexibilité : | 25 mm |
| Résistance à l'acétone : | > 300 s |

Tableau 2 : Caractéristiques moléculaires des produits B-1 et B-2 selon l'invention

| REF | Polyol a) | m ou m moyen polyol a) | r = $CO_2H/OH$ | $r_1 = (CO_2H)_c/(CO_2H)_b$ | $n_{moy}*a$ moyen (calc) | $n_{moy}*b$ moyen (calc) | a | $n_{moy}$ | Mn Calc (g/mole) | Mn GPC (Dalton) | f moyen/ mole (calc) | DAmmoles/ g (calc) |
|---|---|---|---|---|---|---|---|---|---|---|---|---|
| B-1 | TMP et A-1 | 2,72 | 0,926 | 0,191 | 0,335 | 0,337 | 0,50 | 0,672 | 599 | 1200 | 3,20 | 5,34 |
| B-2 | TMP | 3,00 | 0,836 | 0,235 | 1,831 | 0,885 | 0,67 | 2,716 | 1021 | 1900 | 5,72 | 5,60 |

**METHODES DE MESURE ET DE CARACTERISATION**

Détermination de l'aspect : On observe visuellement le produit à la lumière du jour, à travers un flacon en verre blanc de 60 ml et on distingue si le produit est :

- Limpide : aucune turbidité, il est comparable à l'eau,
- Voilé : ne permettant plus une vision nette à travers le flacon,
- Trouble : flacon opaque, aucune image ne peut être perçue à travers le flacon.

Détermination de la turbidité : C'est le pourcentage de lumière diffusée par rapport à la lumière totale transmise par l'échantillon contenu dans une cuve transparente de 50 ml (60 mm X 40 mm X 20 mm). La mesure est effectuée à l'aide d'un spectro-colorimètre « Colorquest XE»® Hunterlab.

Détermination de la viscosité Noury : On mesure le temps de parcours, dans le liquide à caractériser, d'une bille d'acier soumise à sa gravité. La norme AFNOR XP.T51-213 précise en particulier la géométrie du récipient, le diamètre de la bille (2 mm) et le parcours de la bille (104 mm). Dans ces conditions, la viscosité dynamique est proportionnelle au temps de parcours de la bille avec 1 seconde correspondant 0,1 Pa.s.

Détermination de l'indice d'acide (IA) : On exprime l'acidité du produit à caractériser en milligrammes de potasse équivalente par gramme de produit. Pour cela, on effectue un dosage acido-basique dans les conditions suivantes : un poids p de produit (environ exactement 10 grammes) est dissout dans 50 ml d'un mélange toluène / éthanol (2 vol / 1 vol). Après dissolution complète, on titre avec une solution de potasse de normalité N (environ exactement 0,1 N). Le point équivalent est détecté par une électrode combinée asservissant une burette automatique (appareil de titration automatique « 716 DMS Titrino »® Metrohm) délivrant le volume équivalent $V_E$. Après réalisation d'un essai à blanc (50 ml du mélange toluène / éthanol seul) qui permet de déterminer le volume équivalent $V_B$, on calcule l'indice d'acide (IA) par la formule suivante :

$$IA = (V_E - V_B)*N*56,1 / p$$

avec $V_E$ et $V_B$ en ml, N en mole/l et p en gramme.

Détermination de l'indice d'hydroxyle (IOH) : On exprime l'indice d'hydroxyle du produit à caractériser en milligrammes de potasse équivalente par gramme de produit. Pour cela, on effectue un dosage acido-basique en retour après acétylation dans les conditions suivantes : un poids p de produit (environ exactement 10 grammes) est dissout dans exactement 10 ml d'un mélange acétylant (acide para-toluène sulfonique monohydrate : 10 g ; anhydride acétique : 60 g ; acétate d'éthyle : 500 g). On fait réagir en erlen fermé pendant 30 minutes sous agitation à 50°C. On refroidit à température ambiante puis on hydrolyse l'anhydride acétique en excès par l'ajout de 2 ml d'eau qu'on laisse réagir pendant 2 minutes à température ambiante, puis par l'ajout de 10 ml de solution hydrolysante [pyridine / eau] (3 vol / 2 vol) qu'on laisse réagir pendant 5 minutes à température ambiante. On ajoute alors 60 ml de solvant [butanol / toluène] (2 vol / 1 vol). Après homogénéisation, on titre avec une solution de potasse de normalité N (environ exactement 0,5 N). Le point équivalent est détecté par une électrode combinée asservissant une burette automatique (appareil de titration automatique « 716 DMS Titrino »® Metrohm) délivrant le volume équivalent $V_E$. Après réalisation d'un essai à blanc (10 ml du mélange acétylant + 2 ml d'eau + 10 ml de solution hydrolysante + 60 ml de solvant) qui permet de déterminer le volume équivalent $V_B$, on calcule l'indice d'hydroxyle (IOH) par les formules suivantes :

$$IOH^* = (V_B - V_E)*N*56,1 / p$$

avec $V_E$ et $V_B$ en ml, N en mole/l et p en gramme. IOH* : indice d'hydroxyle apparent

$$IOH = IOH^* + IA$$

Détermination de la réactivité : La formulation est appliquée en film de 12 $\mu$m sur une carte contraste (« Penoparc charts form 1B »® Leneta), puis est réticulé à l'aide d'une lampe Fusion Hg à 120 W/cm. On mesure la vitesse de passage minimum nécessaire (en m/min) pour obtenir un film sec au toucher.

Pour les tests suivants de dureté, flexibilité et de résistance à l'acétone, les films photoréticulés sont laissés en salle climatisée (T = 23°C) pendant 24 heures après réticulation et avant les mesures.

Détermination de la dureté Persoz selon norme ISO 1522 : La formulation à examiner est appliquée en film de 100 $\mu$m sur une plaque en verre et réticulée par une lampe Fusion Hg 120 W/cm à une vitesse de 8 m/min.

On mesure le nombre d'oscillations avant l'amortissement des oscillations (passage de 12° à 4° d'amplitude) d'un pendule au contact de la plaque de verre revêtue d'après la norme ISO 1522.

Détermination de la flexibilité : La formulation est appliquée en film de 100 $\mu$m sur une plaque en acier lisse de 25/10 mm d'épaisseur (D-46® Q-Panel), puis réticulé par une lampe Fusion Hg 120 W/cm à une vitesse de 8 m/min.

On courbe la plaque revêtue sur des mandrins cylindriques d'après la norme ISO 1519. On exprime le résultat par la valeur (en mm) du rayon de courbure le plus faible qu'on peut infliger au revêtement sans qu'il ne se fissure, ni se décolle du support.

Détermination de la résistance à l'acétone : La formulation est appliquée en film de 12 $\mu$m sur une plaque en verre, puis réticulée par une lampe Fusion Hg 120 W/cm à une vitesse de 8 m/min. On frotte le revêtement avec un chiffon imbibé d'acétone. Le résultat est le temps (exprimé en seconde) au-delà duquel le film se décolle et/ou se désagrège.

Masse moléculaire moyenne en nombre Mn :

Mn calc : Mn théorique calculée à partir de $n_{MOY}*a$ et $n_{MOY}*b$ selon méthode précisée dans la description, en particulier pour z = 2 :

$$\text{Mn calc} = M[R(OR_1)_m] + a*n_{MOY}*M[A] + b*n_{MOY}*M[B] \text{ (en g / mole)}$$

Avec :

- Terme constant : $M[R(OR_1)_m] = M[R(OH)_m] + 54*m$
- Motif A : $M[A] = M[R(OH)_m] + 54*(m - 1)$
- Motif B : $M[B] = M[R(OH)_m] + M(R2) + 54*(m - 1)$

Mn mesurée : mesurée par GPC dans le THF comme solvant et Mn exprimée en équivalents polystyrène sur colonnes calibrées avec étalons polystyrène.

Fonctionnalité moyenne en nombre du produit en acrylates (voir description) :

Densité en acrylates DA : à partir de f telle que définie ci-dessus en divisant par Mn calculée (voir définition dans description).

$$\text{DA} = 1000*f/Mn.$$

**Revendications**

1. Produit acrylé multifonctionnel, en particulier oligomère acrylé multifonctionnel, **caractérisé en ce qu'**il a une fonctionnalité moyenne en nombre f supérieure à 2,1, de préférence d'au moins 2,5 et plus préférentiellement de 2,75 à 20 et encore plus préférentiellement de 3 à 14 groupements acrylates par mole dudit produit et en particulier avec une densité desdits groupements acrylates DA allant de 2 à 12 mmoles par g dudit produit, ledit produit étant le produit de réaction par estérification et par éthérification par réaction d'addition de Michael, entre :

    a) un polyol $R(OH)_m$ ou un mélange de polyols $R(OH)_m$, de fonctionnalité m d'au moins 3, de préférence de 3 à 6, plus préférentiellement de 4 à 6 pour un seul polyol présent et une fonctionnalité OH moyenne en nombre supérieure à 2,1, de préférence supérieure à 2,3, plus préférentiellement d'au moins 2,5 et jusqu'à 6 pour un mélange desdits polyols et
    b) l'acide acrylique représenté par $R_1OH$,
    ladite réaction entre a) et b) ayant lieu en présence de c) au moins un anhydride carboxylique cyclique ou de sa forme polyacide carboxylique $R_2(CO_2H)z$, de fonctionnalité z en groupements carboxy ($-CO_2H$) d'au moins 2 et allant jusqu'à 4, de préférence de 2 à 3, plus préférentiellement 2 avec :

    - le rapport $r_1$ en nombre de groupements carboxy dudit anhydride c) par rapport à ceux de b) acide acrylique, $r_1 = (CO_2H)_c/(CO_2H)_b$ allant de 0,01 à 0,4, de préférence de 0,05 à 0,35, plus préférentiellement de 0,1 à 0,3
    - les groupements carboxy étant globalement en défaut par rapport aux groupements hydroxy dudit polyol

a), avec r = $CO_2H/OH < 1$, en particulier inférieur ou égal à 0,97

ledit produit acrylé comprenant dans sa composition à la fois :

- des unités A) d'oligoéther-ester acrylate issues de la réaction de a) et de b), formées par une réaction d'addition de Michael :

- des groupements OH dudit polyol a) ou
- de groupements OH d'acrylates partiels hydroxylés formés, sur l'insaturation de l'acide acrylique b) ou sur l'insaturation d'un des acrylates formés par estérification par b) et estérification simultanée par b) dudit polyol a) et desdits acrylates partiels hydroxylés ou (estérification simultannée) par les groupements carboxy de l'adduit carboxylé de Michael formé entre a) et b) et

- des unités B) oligoesters acrylates issues de c) par réaction d'estérification par ledit anhydride ou par sa forme polyacide c) dudit polyol a) ou desdits acrylates partiels hydroxylés ou des éther-ester acrylates hydroxylés formés et ledit produit acrylé étant un mélange de produits acrylés comprenant au moins un produit acrylé p1 associant chimiquement dans sa structure moléculaire les deux types d'unités A) et B) comme définies ci-dessus.

2. Produit selon la revendication 1, **caractérisé en ce que** sa composition globale est représentée par la formule moyenne générale (I) suivante :

$$R_1O-[[A]_a-[B]_b]_n-R(OR_1)_{m-1} \qquad (I)$$

avec a et b représentant la fraction molaire moyenne de chaque unité A) et B) par unité moyenne globale dudit produit et avec a + b = 1 et a/b allant de 0,15 à 22, de préférence de 0,5 à 10, plus préférentiellement de 1 à 5, n étant le nombre d'unités globales répétitives, avec n moyen par mole de produit $n_{moy}$ allant de 0,2 à 10, de préférence de 0,35 à 8, plus préférentiellement de 0,35 à 6 et encore plus préférentiellement de 0,4 à 2,5.

3. Produit selon la revendication 1 ou 2, **caractérisé en ce qu'**il comprend ledit produit p1 et que ledit produit p1 a une structure moléculaire définie selon formule générale (II) suivante :

$$(R_1O)_{m-1}-R-[-[O-CH_2CH_2-CO_2-R(OR_1)_{m-2}]_a \qquad [O_2C-R_2(CO_2X)_{z-2}-CO2-R(OR_1)_{m-2}]_b]_n-OR_1 \qquad (II)$$

et avec présence d'au moins 4 produits de n différent, correspondant à n = 0 et n = 1, n = 2 et n = 3 avec :

- $R_1$ étant le radical acryloyle, R étant le radical résiduel dudit polyol $R(OH)_m$ ou représentant un radical moyen d'un mélange de polyols,
- $R_2$ étant le résidu de valence z dudit anhydride ou sa forme polyacide carboxylique et X étant $-R(OR_1)_{m-1}$ où X pouvant être essentiellement, c'est-à-dire à plus de 95% $-R(OR_1)_{m-1}$ et le reste (moins de 5%) de X étant H, en particulier avec un indice d'acide ne dépassant pas 15 mg KOH/g, plus particulièrement ne dépassant pas 10 mg KOH/g
- n étant le nombre d'unités répétitives et
- a et b étant les fractions molaires respectives des unités particulières dans l'unité répétitive globale avec le rapport a/b allant de 0,15 à 22, de préférence de 0,5 à 10, plus préférentiellement de 1 à 5.

4. Produit selon l'une des revendications 1 à 3, **caractérisé en ce qu'**il comprend en plus dudit produit p1, le produit p2 oligoéther-ester acrylate à base d'unités A) de formule générale (III) suivante :

$$(R_1O)-[-R[OR_1]_{m-2}-O-(C=O)-CH_2-CH_2-O-]_n-R-(OR_1)_{m-1} \qquad (III)$$

avec présence d'au moins 4 produits de n différent et correspondant à n = 0 et n = 1, n = 2 et n = 3 et n étant le nombre d'unités répétitives.

5. Produit selon l'une des revendications 1 à 4, **caractérisé en ce qu'**il comprend en plus dudit produit p1, le produit p3 oligoester acrylate de formule générale (IV) suivante :

$$(R_1O)\text{-}[\text{-}R[OR_1]_{m-2}\text{-}O\text{-}(C=O)\text{-}R2(C=O)O\text{-}]_n\text{-}R\text{-}(OR_1)_{m-1} \qquad (IV)$$

avec présence d'au moins 4 produits de n différent et correspondant à n = 0 et n = 1, n = 2 et n = 3 et n étant le nombre d'unités répétitives.

6. Produit selon la revendication 5, **caractérisé en ce qu'**il comprend un produit p2 tel que défini selon la formule (III) de la revendication 4, ledit produit p1 est tel que défini selon la formule (II) de la revendication 3 et que les 3 produits p1, p2 et p3 ainsi définis, comprennent chacun au moins un cinquième produit correspondant à n = 4 et en option un produit supplémentaire correspondant à n = 5.

7. Produit selon l'une des revendications 1 à 6, **caractérisé en ce que** ledit produit p1 est de formule générale suivante (V) :

$$(R_1O)\text{-}[\text{-}R[OR_1]_{m-2}\text{-}O_2C\text{-}CH_2\text{-}CH_2\text{-}O\text{-}]_n\text{-}R\text{-}(OR_1)_{m-2}\text{-}O_2C\text{-}R_2(CO2\text{-}X_1)_{z-2}\text{-}CO_2\text{-}X_2 \qquad (V)$$

avec $R_2$ étant le radical de valence z correspondant audit anhydride carboxylique ou audit acide polycarboxylique et $X_1$ et $X_2$ pouvant être identiques ou différents et choisis parmi :

- -$R(OR_1)_{m-1}$ ou
- -$R(OR_1)_{m-2}$-[O-$CH_2$-$CH_2$-$CO_2$-$R(OR_1)_{m-2}]_n$-$(OR_1)$ ou
- en partie H ou
- en partie résidu d'un agent bloquant réactif, en particulier monofonctionnel réactif avec le groupement carboxy.

8. Produit selon l'une des revendications 1 à 7, **caractérisé en ce que** la distribution moléculaire globale en n dudit produit est telle qu'elle représente au moins 80% en poids pour n allant de 0 à 4 et pas plus de 20% en poids de ladite distribution pour n étant supérieur à 4, de préférence avec une masse moyenne en nombre Mn dudit produit mesurée par GPC dans le THF et exprimée en équivalents polystyrène allant de 500 à 10000 et plus préférentiellement de 600 à 6000.

9. Produit selon l'une des revendications 1 à 8, **caractérisé en ce que** ledit polyol a) a une fonctionnalité m pour polyol a) seul, d'au moins 3 et que ledit produit comprend des produits p2 oligoéther-ester acrylates linéaires selon la formule générale (III) et en plus au moins un produit oligoéther-ester acrylate de structure ramifiée (ou à chaîne ramifiée).

10. Produit acrylé, **caractérisé en ce qu'**il est susceptible d'être obtenu par réactions simultanées ou successives et alternées entre a) un polyol $R(OH)_m$ ou mélange de polyols $R(OH)_m$, de fonctionnalité m d'au moins 3, de préférence de 3 à 6, plus préférentiellement de 4 à 6, pour un polyol a) seul ou de fonctionnalité moyenne en nombre (sur m) supérieure à 2,1, de préférence supérieure à 2,3, plus préférentiellement d'au moins 2,5 et jusqu'à 6 pour un mélange de polyols $R(OH)_m$ et b) l'acide acrylique $(R_1OH)$ en défaut par rapport à a) et en présence de c) au moins un anhydride carboxylique cyclique ou de sa forme polyacide de fonctionnalité z en groupements carboxy allant de 2 à 4, de préférence de 2 à 3, plus préférentiellement de 2, avec un rapport global r = $CO_2H/OH$ inférieur à 1, en particulier supérieur à $[(m-1)*(r_1+1)]/[m*((r_1/2)+1)]$ et allant jusqu'à 0,97, de préférence supérieur à $1,05*[(m-1)*(r_1+1)]/[m*((r_1/2)+1)]$ et allant jusqu'à 0,97 et plus préférentiellement supérieur à $1,10*[(m-1)*(r_1+1)]/[m*((r_1/2)+1)]$ et allant jusqu'à 0,97, avec $r_1$ étant le rapport des groupements carboxy de c) sur les groupements carboxy de b) acide acrylique $r_1 = (carboxy)_c/(carboxy)_b$, plus particulièrement pour z = 2.

11. Produit selon l'une des revendications 1 à 10, **caractérisé en ce que** ledit polyol a) est sélectionné parmi des monomères polyols et/ou des oligomères polyols avec Mn pour oligomères polyols ne dépassant pas 700, de préférence ne dépassant pas 600.

12. Produit selon la revendication 11, **caractérisé en ce que** ledit polyol est un monomère polyol et sélectionné parmi : diéthylène glycol, triéthylène glycol, tetraéthylène glycol, propylène glycol, dipropylène glycol, tripropylène glycol, tetrapropylène glycol, butane diol, néopentyl glycol, hexane diol, isosorbide, glycérol, triméthylol propane, pentaérythritol, ditriméthylol propane, érythritol, xylitol, dipentaérythritol, sorbitol, y compris les dérivés alcoxylés des polyols cités.

13. Produit selon la revendication 11, **caractérisé en ce que** ledit polyol est un oligomère polyol parmi polyéther polyols, polyester polyols, oligomères acryliques hydroxylés en option alcoxylés et en particulier qu'il est présent en mélange avec au moins un autre polyol a), de préférence un polyol monomère tel que défini selon la revendication 12.

14. Produit selon l'une des revendications 1 à 13, **caractérisé en ce qu'**en plus dudit polyol a) de fonctionnalité d'au moins 3, est présent un deuxième polyol différent du premier de fonctionnalité d'au moins 2, de préférence ce deuxième polyol étant un oligoester diol, plus préférentiellement comprenant comme composant dans son unité répétitive ledit anhydride c) ou sa forme polyacide.

15. Produit selon l'une des revendications 1 à 14, **caractérisé en ce que** ledit anhydride polycarboxylique c) ou sa forme polyacide ou isomère de ce dernier est choisi parmi les anhydrides polycarboxyliques ou polyacides carboxyliques aromatiques ou cycloaliphatiques ou aliphatiques, avec les structures cycloaliphatiques ou aliphatiques pouvant être insaturées.

16. Produit selon la revendication 15, **caractérisé en ce que** ledit anhydride polycarboxylique ou polyacide ou ledit isomère de ce dernier est aromatique et en particulier sélectionné parmi : l'anhydride phtalique (o-), le diacide iso ou téréphtalique, anhydride ou diacide naphténique, anhydride ou polyacide trimellitique, anhydride pyromellitique ou tetra-acide pyromellitique, de préférence ledit anhydride ou polyacide ayant une fonctionnalité de 2.

17. Produit selon la revendication 15, **caractérisé en ce que** ledit anhydride ou sa forme polyacide est sélectionné parmi les anhydrides et diacides correspondants cycloaliphatiques, en particulier parmi l'anhydride et diacide tetrahydrophtalique, anhydride et diacide dihydrophtalique, l'anhydride et diacide nadique (bicyclo (2,2,1) hept-5-ène 2, 3 dicarboxylique) ou l'anhydride et diacide cyclohexane dicarboxylique.

18. Produit selon la revendication 15, **caractérisé en ce que** ledit anhydride ou acide est aliphatique et en particulier choisi parmi : anhydride et diacide maléique, diacide fumarique, anhydride et diacide itaconique, anhydride et diacide succinique.

19. Produit selon la revendication 15, **caractérisé en ce que** ledit anhydride ou acide est choisi parmi : phtalique (o-), le diacide iso ou téréphtalique, l'anhydride et diacide tetrahydrophtalique, l'anhydride et diacide dihydrophtalique, l'anhydride et diacide nadique, l'anhydride et diacide maléique, diacide fumarique, l'anhydride et diacide itaconique, l'anhydride et diacide succinique.

20. Produit selon l'une des revendications 1 à 19, **caractérisé en ce que** ledit anhydride ou polyacide est un mélange d'au moins deux anhydrides et/ou polyacides c).

21. Procédé de préparation d'un produit tel que défini selon l'une des revendications 1 à 20, **caractérisé en ce qu'**il comprend :

i) le mélange dans un réacteur dudit polyol a) de l'acide acrylique b), dudit anhydride ou dudit polyacide c) et dans des proportions telles que le rapport molaire global $r = CO_2H/OH$ soit inférieur à 1 et en particulier supérieur à $[(m-1)*(r_1+1)]/[m*((r_1/2)+1)]$ et allant jusqu'à 0,97, de préférence supérieure à $1,05*[(m-1)*(r_1+1)]/[m*((r_1/2)+1)]$ et allant jusqu'à 0,97 et plus préférentiellement supérieure à $1,10*[(m-1)*(r_1+1)]/[m*((r_1/2)+1)]$ et allant jusqu'à 0,97, avec $r_1 = (CO_2H)_c/(CO_2H)_b$ étant dans la plage 0,01 à 0,4, de préférence de 0,05 à 0,35 et plus préférentiellement de 0,1 à 0,3, plus particulièrement pour z = 2 et en présence d'un catalyseur acide d'estérification et d'un solvant formant un azéotrope avec l'eau pour former le mélange réactionnel, suivi de
ii) la mise sous reflux dudit mélange réactionnel, avec réactions simultanées ou successives et alternées d'estérification, par réaction de l'acide acrylique b) et /ou dudit anhydride ou polyacide c) avec un hydroxy dudit polyol a) avec formation d'esters acrylates hydroxylés et d'éthérification par réaction d'addition de Michael d'un hydroxy dudit polyol ou dudit acrylate hydroxylé formé sur un groupement acrylate et/ou l'acide acrylique b) et d'estérification des groupements hydroxy dudit polyol et desdits acrylates hydroxylés par ledit anhydride ou diacide et élimination progressive de l'eau d'estérification, avec
iii) poursuite de la réaction jusqu'à consommation complète des fonctions OH ($I_{OH}$ < 20 mg KOH/g) par réactions d'addition de Michael et d'estérification avec ledit acide acrylique b) et ledit anhydride ou diacide c)
iv) neutralisation dudit catalyseur acide avant récupération du produit final, par élimination dudit solvant, sans aucune autre étape spécifique de purification requise.

22. Composition réticulable, **caractérisée en ce qu'**elle comprend au moins un produit tel que défini selon l'une des revendications 1 à 20 ou obtenu par un procédé tel que défini selon la revendication 21.

**23.** Composition selon la revendication 22, **caractérisée en ce que** dans le cas où Mn calculée dudit produit est supérieure à 1000, de préférence supérieure à 1500, elle comprend en plus dudit produit, au moins un diluant réactif, sélectionné parmi les monomères acryliques de préférence multifonctionnels.

**24.** Composition selon la revendication 22 ou 23, **caractérisée en ce qu'**elle est une composition réticulable, de préférence par rayonnement, en particulier par rayonnement UV en présence d'un système photoamorceur ou par faisceau d'électrons (EB) en l'absence de système photoamorceur et/ou par un système d'amorçage radicalaire thermique, en particulier par système d'amorçage peroxyde (P-cure) et/ou par addition de Michael (M-cure) ou par un système mixte, en particulier par réticulation duale (dual cure) et plus particulièrement avec présence d'au moins deux des systèmes de réticulation précités.

**25.** Composition selon l'une des revendications 22 à 24, **caractérisée en ce qu'**il s'agit d'une composition de revêtements pigmentés ou non pigmentés, de préférence parmi peinture, vernis, encre, adhésif ou d'une composition de moulage ou d'une composition d'étanchéité ou d'une composition de composite ou d'une composition de scellement chimique ou d'une composition pour impression 3D ou d'une composition pour objets en 3D couche par couche.

**26.** Utilisation d'un produit tel que défini selon l'une des revendications 1 à 20 ou obtenu par un procédé tel que défini selon la revendication 21, dans des compositions réticulables, en particulier dans des compositions réticulables présentant un faible taux de retrait, de préférence dans des compositions réticulables de revêtements.

**27.** Utilisation selon la revendication 26, **caractérisée en ce qu'**elle s'applique à des compositions de revêtements pigmentés ou non pigmentés, de préférence parmi peinture, vernis, encre, adhésif ou d'une composition de moulage ou d'une composition d'étanchéité ou d'une composition de composite ou d'une composition de scellement chimique ou d'une composition pour impression 3D ou d'une composition pour objets en 3D couche par couche.

**28.** Produit fini réticulé, en particulier choisi parmi les revêtements pigmentés ou non pigmentés réticulés, de préférence parmi les peintures, vernis, encres, adhésifs ou choisi parmi les pièces moulées, les joints d'étanchéité, les composites, les scellements chimiques, impressions 3D ou objets en 3D couche par couche, **caractérisé en ce qu'**il résulte de l'utilisation d'au moins un produit tel que défini selon l'une des revendications 1 à 20 ou obtenu par un procédé tel que défini selon la revendication 21 ou de la réticulation d'une composition réticulable telle que définie selon l'une des revendications 22 à 25.

**Patentansprüche**

**1.** Multifunktionelles acryliertes Produkt, insbesondere multifunktionelles acryliertes Oligomer, **dadurch gekennzeichnet, dass** dieses eine zahlenmittlere Funktionalität f von mehr als 2, 1, vorzugsweise mindestens 2,5 und bevorzugter von 2,75 bis 20 oder noch bevorzugter von 3 bis 14 Acrylatgruppen pro Mol des Produkts aufweist, und insbesondere mit einer Dichte der Acrylatgruppen DA von 2 bis 12 mmol pro g des Produkts, wobei das Produkt das Reaktionsprodukt durch Veresterung und durch Veretherung durch eine Michael-Additionsreaktion ist zwischen:

a) einem Polyol $R(OH)_m$ oder einer Mischung von Polyolen $R(OH)_m$, mit einer Funktionalität m von mindestens 3, vorzugsweise von 3 bis 6, bevorzugter von 4 bis 6 für ein einzelnes vorliegendes Polyol, und einer zahlenmittleren OH-Funktionalität von mehr als 2,1, vorzugsweise mehr als 2,3, bevorzugter mindestens 2,5 und bis 6 für eine Mischung der Polyole, und
b) Acrylsäure, die durch $R_1OH$ dargestellt wird,

wobei die Reaktion stattfindet zwischen a) und b) in Anwesenheit von c) mindestens einem cyclischen Carbonsäureanhydrid oder seiner Polycarbonsäure-Form $R_2(CO_2H)z$, mit einer Carboxygruppen- (-$CO_2H$-) Funktionalität z von mindestens 2 und bis 4, vorzugsweise von 2 bis 3, bevorzugter 2, wobei:

- das Zahlenverhältnis $r_1$ von Carboxygruppen des Anhydrids c) im Verhältnis zu jenen von b), der Acrylsäure, $r_1 = (CO_2H)_c/(CO_2H)_b$, von 0,01 bis 0,4, vorzugsweise von 0,05 bis 0,35, bevorzugter von 0,1 bis 0,3 beträgt,
- die Carboxygruppen allgemein weniger sind im Verhältnis zu den Hydroxygruppen des Polyols a), wobei r = $CO_2H/OH < 1$, insbesondere kleiner oder gleich 0,97,

das acrylierte Produkt in seiner Zusammensetzung gleichzeitig umfasst:

- Oligoether-Acrylatester-Einheiten A) aus der Reaktion von a) und von b), die durch eine Michael-Additionsreaktion gebildet werden:
- von Gruppen OH des Polyols a) oder
- von Gruppen OH hydroxylierter partieller Acrylate, die gebildet werden an der Unsättigung der Acrylsäure b) oder an der Unsättigung eines der Acrylate, die gebildet werden durch Veresterung durch b) und gleichzeitige Veresterung durch b) des Polyols a) und der hydroxylierten partiellen Acrylate oder (gleichzeitige Veresterung) durch die Carboxygruppen des carboxylierten Michael-Addukts, der zwischen a) und b), und
- Oligoesteracrylat-Einheiten B), die stammen aus c) durch eine Veresterungsreaktion durch das Anhydrid oder durch seine Polysäure-Form c) des Polyols a) oder der hydroxylierten partiellen Acrylate oder der gebildeten hydroxylierten Acrylatesher-Ether,

und das acrylierte Produkt eine Mischung acrylierter Produkte ist, umfassend mindestens ein acryliertes Produkt p1, das in seiner Molekülstruktur die beiden Typen von Einheiten A) und B), wie oben definiert, chemisch assoziiert.

2. Produkt nach Anspruch 1,
**dadurch gekennzeichnet, dass** seine allgemeine Zusammensetzung durch die folgende allgemeine mittlere Formel (I) dargestellt ist:

$$R_1O\text{-}[[A]_a\text{-}[B]_b]_n\text{-}R(OR_1)_{m-1} \qquad (I)$$

wobei a und b die mittlere Molfraktion jeder Einheit A) und B) pro allgemeine mittlere Einheit des Produkts darstellen, und wobei a + b = 1 und a/b von 0,15 bis 22, vorzugsweise von 0,5 bis 10, bevorzugter von 1 bis 5 beträgt, n die Anzahl allgemeiner Wiederholungseinheiten ist, wobei der Mittelwert von n pro Mol des Produkts $n_{moy}$ von 0,2 bis 10, vorzugsweise von 0,35 bis 8, bevorzugter von 0,35 bis 6 und noch bevorzugter von 0,4 bis 2,5 beträgt.

3. Produkt nach Anspruch 1 oder 2,
**dadurch gekennzeichnet, dass** dieses das Produkt p1 umfasst, und dass das Produkt p1 eine Molekularstruktur aufweist, die gemäß der folgenden allgemeinen Formel (II) definiert ist:

$$(R_1O)_{m-1}\text{-}R\text{-}[\text{-}[O\text{-}CH_2CH_2\text{-}CO_2\text{-}R(OR_1)_{m-2}]_a[O_2C\text{-}R2(CO_2X)_{z-2}CO2\text{-}R(OR_1)_{m-2}]_b]_n\text{-}OR_1 \qquad (II)$$

und mit dem Vorliegen von mindestens 4 verschiedenen Produkten von n, die n = 0, n = 1, n = 2 und n = 3 entsprechen, wobei:

- $R_1$ ein Acryloylrest ist, wobei R der Rest des Polyols $R(OH)_m$ ist oder einen mittleren Rest einer Mischung von Polyolen darstellt,
- $R_2$ ein Rest mit der Wertigkeit z des Anhydrids oder seiner Polycarbonsäure-Form ist, und X $-R(OR_1)_{m-1}$ ist, oder X im Wesentlichen, das heißt, zu mehr als 95 %, $-R(OR_1)_{m-1}$ sein kann und der Rest (mindestens 5 %) von X die Bedeutung H hat, insbesondere mit einem Säureindex nicht über 15 mg KOH/g, insbesondere nicht über 10 mg KOH/g,
- n die Anzahl von Wiederholungseinheiten ist, und
- a und b die jeweiligen Molfraktionen der bestimmten Einheiten in der allgemeinen Wiederholungseinheit sind, wobei das Verhältnis a/b von 0,15 bis 22, vorzugsweise von 0,5 bis 10, bevorzugter von 1 bis 5 beträgt.

4. Produkt nach einem der Ansprüche 1 bis 3,
**dadurch gekennzeichnet, dass** dieses zusätzlich zu dem Produkt p1 das Produkt p2 Oligoether-Acrylester auf der Basis der Einheiten A) mit der folgenden allgemeinen Formel (III) umfasst:

$$(R_1O)\text{-}[\text{-}R[OR_1]_{m-2}\text{-}O\text{-}(C=O)\text{-}CH_2\text{-}CH_2\text{-}O\text{-}]_n\text{-}R\text{-}(OR_1)_{m-1} \qquad (III)$$

mit dem Vorliegen von mindestens 4 Produkten von n, die verschieden sind und n = 0, n = 1, n = 2 und n = 3 entsprechen, und wobei n die Anzahl von Wiederholungseinheiten ist.

5. Produkt nach einem der Ansprüche 1 bis 4,
**dadurch gekennzeichnet, dass** dieses zusätzlich zu dem Produkt p1 das Produkt p3 Oligoacrylatester mit der folgenden allgemeinen Formel (IV) umfasst:

$$(R_1O)\text{-}[R[OR_1]_{m\text{-}2}\text{-}O\text{-}(C=O)\text{-}R2(C=O)O\text{-}]_n\text{-}R\text{-}(OR_1)_{m\text{-}1} \qquad (IV)$$

mit dem Vorliegen von mindestens 4 Produkten von n, die verschieden sind und n = 0, n = 1, n = 2 und n = 3 entsprechen, und wobei n die Anzahl von Wiederholungseinheiten ist.

6. Produkt nach Anspruch 5,
**dadurch gekennzeichnet, dass** dieses ein Produkt p2, wie gemäß der Formel (III) von Anspruch 4 definiert, umfasst, wobei das Produkt p1 wie gemäß der Formel (II) von Anspruch 3 definiert ist, und dass die 3 so definierten Produkte p1, p2 und p3 jeweils mindestens ein fünftes Produkt, das n = 4 entspricht, und gegebenenfalls ein ergänzendes Produkt, das n = 5 entspricht, umfassen.

7. Produkt nach einem der Ansprüche 1 bis 6,
**dadurch gekennzeichnet, dass** das Produkt p1 die folgende allgemeine Formel (V) aufweist:

$$(R_1O)\text{-}[R[OR_1]_{m\text{-}2}\text{-}O_2C\text{-}CH_2\text{-}CH_2\text{-}O\text{-}]_n\text{-}R\text{-}(OR_1)_{m\text{-}2}O_2CR_2(CO2\text{-}X_1)_{z\text{-}2}\text{-}CO_2\text{-}X_2 \qquad (V)$$

wobei $R_2$ ein Rest mit der Wertigkeit z ist, der dem Carbonsäureanhydrid oder der Polycarbonsäure entspricht, und
$X_1$ und $X_2$ identisch oder verschieden sein können und ausgewählt sind aus:

- $R(OR_1]_{m\text{-}1}$ oder
- $\text{-}R(OR_1)_{m\text{-}2}\text{-}[O\text{-}CH_2\text{-}CH_2\text{-}CO_2\text{-}R(OR_1)_{m\text{-}2}]_n\text{-}(OR_1)$ oder
- teilweise H oder
- teilweise einem Rest eines reaktiven, insbesondere monofunktionellen reaktiven Blockierungsmittels mit der Carboxygruppe.

8. Produkt nach einem der Ansprüche 1 bis 7,
**dadurch gekennzeichnet, dass** die allgemeine molekulare Verteilung von n des Produkts derart ist, dass sie mindestens 80 Gew.-% für n von 0 bis 4 darstellt, und nicht mehr als 20 Gew.-% der Verteilung größer als 4 sind, vorzugsweise mit einer zahlenmittleren Masse Mn des Produkts, gemessen durch GPC in THF, und ausgedrückt in Polystyrol-Äquivalenten, von 500 bis 10000 und bevorzugter von 600 bis 6000.

9. Produkt nach einem der Ansprüche 1 bis 8,
**dadurch gekennzeichnet, dass** das Polyol a) eine Funktionalität m für Polyol a) allein von mindestens 3 aufweist, und dass das Produkt Produkte p2 von linearen Oligoether-Acrylatester gemäß der allgemeinen Formel (III) und außerdem mindestens ein Produkt Oligoether-Acrylatester mit verzweigter Struktur (oder mit verzweigter Kette) umfasst.

10. Acryliertes Produkt,
**dadurch gekennzeichnet, dass** dieses erhalten werden kann durch gleichzeitige oder aufeinanderfolgende und abwechselnde Reaktionen zwischen a) einem Polyol $R(OH)_m$ oder einer Mischung von Polyolen $R(OH)_m$ mit einer Funktionalität m von mindestens 3, vorzugsweise von 3 bis 6, bevorzugter von 4 bis 6, für ein Polyol a) allein oder mit einer zahlenmittleren Funktionalität (von m) von mehr als 2,1, vorzugsweise mehr als 2,3, bevorzugter mindestens 2,5 und bis 6 für eine Mischung von Polyolen $R(OH)_m$, und b) Acrylsäure $(R_1OH)$ weniger im Verhältnis zu a) und in Anwesenheit von c) mindestens einem cyclischen Carbonsäureanhydrid oder seiner Polysäure-Form mit einer Funktionalität z von Carboxygruppen von 2 bis 4, vorzugsweise von 2 bis 3, bevorzugter von 2, mit einem allgemeinen Verhältnis r = $CO_2H/OH$ von weniger als 1, insbesondere größer als $[(m\text{-}1)*(r_1+1]/[m*((r_1/2)+1)]$ und bis 0,97, vorzugsweise größer als $1,05*[(m\text{-}1)*(r_1+1]/[m*((r_1/2)+1)]$ und bis 0,97, und bevorzugter größer als $1,10*[(m\text{-}1)*(r_1+1)]/[m*((r_{1/}2)+1)]$ und bis 0,97, wobei n das Verhältnis der Carboxygruppen von c) zu den Carboxygruppen von b) Acrylsäure ist, $r_1$ = $(Carboxy)_c/(Carboxy)_b$, insbesondere für z = 2.

11. Produkt nach einem der Ansprüche 1 bis 10,
**dadurch gekennzeichnet, dass** das Polyol a) aus Polyolmonomeren und/oder Polyololigomeren ausgewählt ist, wobei Mn für Polyololigomere 700 nicht überschreitet, vorzugsweise 600 nicht überschreitet.

12. Produkt nach Anspruch 11,
**dadurch gekennzeichnet, dass** das Polyol ein Polyolmonomer ist und ausgewählt ist aus: Diethylenglykol, Triethylenglykol, Tetraethylenglykol, Propylenglykol, Dipropylenglykol, Tripropylenglykol, Tetrapropylenglykol, Butandiol, Neopentylglykol, Hexandiol, Isosorbidglycerin, Trimethylolpropan, Pentaerythrit, Ditrimethylolpropan, Erythrit, Xylit, Dipentaerythrit, Sorbit, einschließlich alkoxylierter Derivate der angeführten Polyole.

13. Produkt nach Anspruch 11,
**dadurch gekennzeichnet, dass** das Polyol ein Polyololigomer ist unter Polyetherpolyolen, Polyesterpolyolen, hydroxylierten, gegebenenfalls alkoxylierten Acryloligomeren, und das insbesondere in Mischung mit mindestens einem anderen Polyol a) vorliegt, vorzugsweise einem Monomerpolyol, wie nach Anspruch 12 definiert.

14. Produkt nach einem der Ansprüche 1 bis 13,
**dadurch gekennzeichnet, dass** zusätzlich zu dem Polyol a) mit einer Funktionalität von mindestens 3 ein zweites Polyol, das von dem ersten verschieden ist, mit einer Funktionalität von mindestens 2 vorliegt, wobei vorzugsweise dieses zweite Polyol ein Oligoesterdiol ist, bevorzugter umfassend das Anhydrid c) oder seine Polysäure-Form als Komponente in seiner Wiederholungseinheit.

15. Produkt nach einem der Ansprüche 1 bis 14,
**dadurch gekennzeichnet, dass** das Polycarbonsäureanhydrid c) oder seine Polysäure- oder Isomer-Form dieses Letzteren ausgewählt ist aus Polycarbonsäureanhydriden oder aromatischen oder cycloaliphatischen oder aliphatischen Polycarbonsäuren, wobei die cycloaliphatischen oder aliphatischen Strukturen ungesättigt sein können.

16. Produkt nach Anspruch 15,
**dadurch gekennzeichnet, dass** das Polycarbonsäureanhydrid oder die Polysäure oder das Isomer dieser Letzteren aromatisch und insbesondere ausgewählt ist aus: Phthalsäureanhydrid (o-), Iso- oder Terephthaldisäure, Napthensäureanhydrid oder -disäure, Trimellitsäureanhydrid oder - polysäure, Pyromellitsäureanhydrid oder Pyromellittetrasäure, vorzugsweise dem Anhydrid oder der Polysäure mit einer Funktionalität von 2.

17. Produkt nach Anspruch 15,
**dadurch gekennzeichnet, dass** das Anhydrid oder seine Polysäure-Form ausgewählt ist aus Anhydriden und entsprechenden cycloaliphatischen Disäuren, insbesondere aus Tetrahydrophthalssäureanhydrid und -disäure, Dihydrophthalsäureanhydrid- und -disäure, Nadicsäureanhydrid und -disäure (Bicyclo(2,2,l)hept-5-en-2,3-dicarbonsäure) oder Cyclohexandicarbonsäureanhydrid- oder -disäure.

18. Produkt nach Anspruch 15,
**dadurch gekennzeichnet, dass** das Anhydrid oder die Säure alphatisch ist und insbesondere ausgewählt ist aus: Maleinsäureanhydrid und -disäure, Fumardisäure, Itaconsäureanhydrid und -disäure, Bernsteinsäureanhydrid und -disäure.

19. Produkt nach Anspruch 15,
**dadurch gekennzeichnet, dass** das Anhydrid oder die Säure ausgewählt ist aus: Phthalsäure (o-), Iso- oder Terephthaldisäure, Tetrahydrophthalsäureanhydrid und -disäure, Nadicsäureanhydrid und -disäure, Maleinsäureanhydrid und -disäure, Fumardisäure,
Itaconsäureanhydrid und -disäure, Bernsteinsäureanhydrid und -disäure.

20. Produkt nach einem der Ansprüche 1 bis 19,
**dadurch gekennzeichnet, dass** das Anhydrid oder die Polysäure eine Mischung von mindestens zwei Anhydriden und/oder Polysäuren c) ist.

21. Verfahren zur Herstellung eines Produkts, wie nach einem der Ansprüche 1 bis 20 definiert, **dadurch gekennzeichnet, dass** dieses umfasst:

i) Mischen, in einem Reaktor, des Polyols a) von Acrylsäure b), des Anhydrids oder der Polysäure c) und in solchen Mengen, dass das allgemeine Molverhältnis $r = CO_2H/OH$ kleiner ist als 1 und insbesondere größer als $[(m-1)*(r_1+1)]/[m*((r_1/2)+1)]$ und bis 0,97, vorzugsweise größer als $1,05*[(m-1)*(r_1+1]/[m*((r_1/2)+1)]$ und bis 0,97, und bevorzugter größer als $1,10*[(m-1)*(r_1+1]/[m*((r_1/2)+1)]$ und bis 0,97, wobei $r_1 = (CO_2H)_c/(CO_2H)_b$ im Bereich von 0,01 bis 0,4 liegt, vorzugsweise von 0,05 bis 0,35, bevorzugter von 0,1 bis 0,3, insbesondere für $z = 2$, und in Anwesenheit eines sauren Veresterungskatalysators und eines Lösungsmittels, das ein Azeotrop mit Wasser bildet, um die Reaktionsmischung zu bilden, gefolgt von
ii) Halten der Reaktionsmischung am Rückfluss, mit gleichzeitigen oder aufeinanderfolgenden und abwechselnden Reaktionen einer Veresterung, durch Umsetzen der Acrylsäure b) und/oder des Anhydrids oder der Polysäure c) mit einem Hydroxy des Polyols a) mit der Bildung von hydroxylierten Acrylatestern, und einer Veretherung durch eine Michael-Additionsreaktion eines Hydroxy des Polyols oder des gebildeten hydroxylierten Acrylats mit einer Acrylatgruppe und/oder der Acrylsäure b), und einer Veresterung der Hydroxygruppen des Polyols und der hydroxylierten Acrylate durch das Anhydrid oder die Disäure, und einer progressiven Elminierung von Veresterungswasser, mit

iii) Verfolgen der Reaktion bis zum vollständigen Verbrauch der Funktionen OH ($I_{OH}$ < 20 mg KOH/g) durch Reaktionen einer Michael-Addition und Veresterung mit der Acrylsäure b) und dem Anhydrid oder der Disäure c),
iv) Neutralisieren des sauren Katalysators vor der Gewinnung des Endprodukts durch Eliminieren des Lösungsmittels ohne irgendeinen anderen spezifischen erforderlichen Reinigungsschritt.

**22.** Vernetzbare Zusammensetzung,
**dadurch gekennzeichnet, dass** diese mindestens ein Produkt, wie in einem der Ansprüche 1 bis 20 definiert, oder erhalten durch ein Verfahren, wie in Anspruch 21 definiert, umfasst.

**23.** Zusammensetzung nach Anspruch 22,
**dadurch gekennzeichnet, dass** in dem Fall, wo die berechnete Mn des Produkts größer ist als 1000, vorzugsweise größer als 1500, diese zusätzlich zu dem Produkt mindestens ein reaktives Verdünnungsmittel umfasst, ausgewählt aus vorzugsweise multifunktionellen Acrylmonomeren.

**24.** Zusammensetzung nach Anspruch 22 oder 23,
**dadurch gekennzeichnet, dass** diese eine vernetzbare Zusammensetzung ist, vorzugsweise durch Strahlung, insbesondere durch UV-Strahlung, in Anwesenheit eines Photoinitiatorsystems oder durch einen Elektronenstrahl (EB) in Abwesenheit eines Photoinitiatorsystems und/oder durch ein Radikalsystem mit thermischer Initiierung, insbesondere durch ein Peroxid-Initiierungssystem (P-cure) und/oder durch Michael-Addition (M-cure) oder durch ein gemischtes System, insbesondere durch duale Vernetzung (dual cure) und insbesondere in Anwesenheit von mindestens zwei vorgenannten Vernetzungssystemen.

**25.** Zusammensetzung nach einem der Ansprüche 22 bis 24,
**dadurch gekennzeichnet, dass** es sich um eine Zusammensetzung für pigmentierte oder nichtpigmentierte Beschichtungen handelt, vorzugsweise unter Anstrichen, Lacken, Tinte, Haftmitteln, oder eine Formverbindung oder eine Dichtungszusammensetzung oder eine Verbundstoffzusammensetzung oder eine chemische Versiegelungszusammensetzung oder eine Zusammensetzung für einen 3D Druck oder eine Zusammensetzung für 3D Objekte Schicht für Schicht.

**26.** Verwendung eines Produkts, wie nach einem der Ansprüche 1 bis 20 definiert, oder erhalten durch ein Verfahren, wie nach Anspruch 21 definiert, in vernetzbaren Zusammensetzungen, insbesondere in vernetzbaren Zusammensetzungen, die ein geringes Streckverhältnis aufweisen, vorzugsweise in vernetzbaren Zusammensetzungen für Beschichtungen.

**27.** Verwendung nach Anspruch 26,
**dadurch gekennzeichnet, dass** diese angewendet wird bei Zusammensetzungen von pigmentierten oder nichtpigmentierten Beschichtungen, vorzugsweise unter Anstrichen, Lacken, Tinte, Haftmitteln, oder einer Formverbindung oder einer Dichtungszusammensetzung oder einer Verbundstoffzusammensetzung oder einer chemischen Versiegelungszusammensetzung oder einer Zusammensetzung für einen 3D Druck oder einer Zusammensetzung für 3D Objekte Schicht für Schicht.

**28.** Vernetztes Endprodukt, insbesondere ausgewählt aus pigmentierten oder nicht-pigmentierten Beschichtungen, vorzugsweise unter Anstrichen, Lacken, Tinte, Haftmitteln, oder ausgewählt aus Formstücken, Dichtungen, Verbundstoffen, chemischen Versiegelungen, 3D Druck oder 3D Objekten Schicht für Schicht,
**dadurch gekennzeichnet, dass** dieses durch die Verwendung mindestens eines Produkts, wie nach einem der Ansprüche 1 bis 20 definiert, oder erhalten durch ein Verfahren, wie nach Anspruch 21 definiert, oder durch die Vernetzung einer vernetzbaren Zusammensetzung, wie nach einem der Ansprüche 22 bis 25 definiert, erhalten wird.

**Claims**

**1.** Multifunctional acrylic product, in particular a multifunctional acrylic oligomer, **characterized in that** it has a number-average functionality f greater than 2.1, preferably of at least 2.5 and more preferentially from 2.75 to 20 and even

more preferably from 3 to 14 acrylic groups per mole of said product and in particular with a density of said acrylic groups DA ranging from 2 to 12 mmol per g of said product, said product being the product of reaction by esterification and by etherification, by Michael addition reaction, between:

a) a polyol $R(OH)_m$ or a mixture of polyols $R(OH)_m$, of functionality m of at least 3, preferably of 3 to 6, more preferably of 4 to 6 for a single polyol present and a number-average OH functionality greater than 2.1, preferably greater than 2.3, more preferentially of at least 2.5 and up to 6, for a mixture of said polyols, and
b) the acrylic acid represented by $R_1OH$,

said reaction between a) and b) taking place in the presence of c) at least one cyclic carboxylic anhydride or of its polycarboxylic acid form $R_2(CO_2H)z$, of carboxy group ($-CO_2H$) functionality z of at least 2 and ranging up to 4, preferably from 2 to 3, more preferentially 2, with:

- the ratio $r_1$ of number of carboxy groups of said anhydride c) relative to those of b) acrylic acid, $r_1 = (CO_2H)_c/(CO_2H)_b$ ranging from 0.01 to 0.4, preferably from 0.05 to 0.35 and more preferentially from 0.1 to 0.3,
- the carboxy groups being overall in deficit relative to the hydroxyl groups of said polyol a), with $r = CO_2H/OH < 1$, in particular less than or equal to 0.97

said acrylic product comprising in its composition both:

- units A) of oligoether-ester acrylate that are derived from the reaction of a) and of b), formed by a Michael addition reaction:

- of the OH groups of said polyol a) or
- of OH groups of hydroxylated partial acrylates formed on the unsaturation of the acrylic acid b) or on the unsaturation of one of the acrylates formed by esterification with b) and simultaneous esterification with b) of said polyol a) and of said hydroxylated partial acrylates or (simultaneous esterification) with the carboxy groups of the carboxylated Michael adduct formed between a) and b), and

- units B) of oligoester acrylates derived from c) by a reaction of esterification with said anhydride or with its polyacid form c) of said polyol a) or of said hydroxylated partial acrylates or of the hydroxylated ether-ester acrylates formed,

and said acrylic product being a mixture of acrylic products comprising at least one acrylic product p1 chemically linking, in its molecular structure, the two types of units A) and B) as defined above.

2. Product according to Claim 1, **characterized in that** its overall composition is represented by the following average general formula (I):

$$R_1O\text{-}[[A]_a\text{-}[B]_b]_n\text{-}R(OR_1)_{m-1} \qquad (I)$$

with a and b representing the average mole fraction of each unit A) and B) per overall average unit of said product and with $a + b = 1$ and a/b ranging from 0.15 to 22, preferably from 0.5 to 10, more preferentially from 1 to 5, n being the number of repeat overall units, with average n per mole of product $n_{ave}$ ranging from 0.2 to 10, preferably from 0.35 to 8, more preferentially from 0.35 to 6 and even more preferentially from 0.4 to 2.5.

3. Product according to Claim 1 or 2, **characterized in that** it comprises said product p1 and that said product p1 has a molecular structure defined according to general formula (II) below:

$$(R_1O)_{m-1}\text{-}R\text{-}[\text{-}[O\text{-}CH_2CH_2\text{-}CO_2\text{-}R(OR_1)_{m-2}]_a[O_2C\text{-}R_2(CO_2X)_{z-2}\text{-}CO2\text{-}R(OR_1)_{m-2}]_b]_n\text{-}OR1 \qquad (II)$$

and with the presence of at least four products having a different n, corresponding to n = 0 and n = 1, n = 2 and n = 3 with:

- $R_1$ being the acryloyl radical, R being the residual radical of said polyol $R(OH)_m$ or representing an average radical of a mixture of polyols,
- $R_2$ being the residue of valency z of said anhydride or its polycarboxylic acid form and X being $-R(OR_1)_{m-1}$

with X possibly being essentially, i.e. more than 95%, $-R(OR_1)_{m-1}$ and the rest (less than 5%) of X being H, in particular with an acid number not exceeding 15 mg KOH/g, more particularly not exceeding 10 mg KOH/g,
- n being the number of repeat units and
- a and b being the respective mole fractions of the particular units in the overall repeat unit with the ratio a/b ranging from 0.15 to 22, preferably from 0.5 to 10, more preferentially from 1 to 5.

4. Product according to one of Claims 1 to 3, **characterized in that** it comprises, in addition to said product p1, the oligoether-ester acrylate product p2 based on units A) of general formula (III) below:

$$(R_1O)-[-R[OR_1]_{m-2}-O-(C=O)-CH_2-CH_2-O-]_n-R-(OR_1)_{m-1} \qquad (III)$$

with the presence of at least four products having a different n and corresponding to n = 0 and n = 1, n = 2 and n = 3 and n being the number of repeat units.

5. Product according to one of Claims 1 to 4, **characterized in that** it comprises, in addition to said product p1, the oligoester acrylate product p3 of general formula (IV) below:

$$(R_1O)-[-R[OR_1]_{m-2}-O-(C=O)-R2(C=O)O-]_n-R-(OR_1)_{m-1} \qquad (IV)$$

with the presence of at least four products having a different n and corresponding to n = 0 and n = 1, n = 2 and n = 3 and n being the number of repeat units.

6. Product according to Claim 5, **characterized in that** it comprises a product p2 as defined according to formula (III) of Claim 4, said product p1 is as defined according to formula (II) of Claim 3 and the three products p1, p2 and p3 thus defined each comprise at least a fifth product corresponding to n = 4 and, as an option, an additional product corresponding to n = 5.

7. Product according to one of Claims 1 to 6, **characterized in that** said product p1 has the following general formula (V):

$$(R_1O)-[-R[OR_1]_{m-2}-O_2C-CH_2-CH_2-O-]_n-R-(OR_1)_{m-2}-O_2C-R_2(CO2-X_1)_{z-2}-CO_2-X_2 \qquad (V)$$

with $R_2$ being the radical having valency z corresponding to said carboxylic anhydride or to said polycarboxylic acid and
$X_1$ and $X_2$ possibly being identical or different and chosen from:

- $-R(OR_1)_{m-1}$ or
- $-R(OR_1)_{m-2}-[O-CH_2-CH_2-CO_2-R(OR_1)_{m-2}]_n-(OR_1)$ or
- in part H or
- in part the residue of a reactive blocking agent, in particular a monofunctional reactive blocking agent, reactive with the carboxy group.

8. Product according to one of Claims 1 to 7, **characterized in that** the overall molecular distribution in terms of n of said product is such that it represents at least 80% by weight for n ranging from 0 to 4 and no more than 20% by weight of said distribution for n being greater than 4, preferably with a number-average weight Mn of said product, measured by GPC in THF and expressed in polystyrene equivalents, ranging from 500 to 10000 and more preferentially from 600 to 6000.

9. Product according to one of Claims 1 to 8, **characterized in that** said polyol a) has a functionality m for polyol a) alone of at least 3 and that said product comprises linear oligoether-ester acrylate products p2 according to general formula (III) and also at least one oligoether-ester acrylate product of branched structure (or comprising a branched chain).

10. Acrylic product, **characterized in that** it can be obtained by simultaneous or successive and alternating reactions between a) a polyol $R(OH)_m$ or a mixture of polyols $R(OH)_m$, having a functionality m of at least 3, preferably from 3 to 6, more preferentially from 4 to 6, for a polyol a) alone, or having a number-average functionality (with respect to m) greater than 2.1, preferably greater than 2.3, more preferentially of at least 2.5 and up to 6 for a mixture of polyols $R(OH)_m$ and b) the acrylic acid $(R_1OH)$ in deficit relative to a), and in the presence of c) at least one cyclic carboxylic anhydride or of its polyacid form having a carboxy group functionality z ranging from 2 to 4, preferably from 2 to 3, more preferentially of 2, with an overall ratio $r = CO_2H/OH$ of less than 1, in particular greater than $[(m-1)*(r_1+1)]/[m*((r_1/2)+1)]$ and ranging up to 0.97, preferably greater than $1.05*[(m-1)*(r_1+1)]/[m*((r_1/2)+1)]$ and ranging up to 0.97 and more preferentially greater than $1.10*[(m-1)*(r_1+1)]/[m*((r_1/2)+1)]$ and ranging up to 0.97, with $r_1$ being the ratio of the carboxy groups of c) to the carboxy groups of b) acrylic acid, $r_1 = (carboxy)_c/(carboxy)_b$, more particularly with $z = 2$.

11. Product according to one of Claims 1 to 10, **characterized in that** said polyol a) is selected from polyol monomers and/or polyol oligomers with Mn for polyol oligomers not exceeding 700, preferably not exceeding 600.

12. Product according to Claim 11, **characterized in that** said polyol is a polyol monomer and selected from: diethylene glycol, triethylene glycol, tetraethylene glycol, propylene glycol, dipropylene glycol, tripropylene glycol, tetrapropylene glycol, butanediol, neopentyl glycol, hexanediol, isosorbide, glycerol, trimethylolpropane, pentaerythritol, ditrimethylolpropane, erythritol, xylitol, dipentaerythritol and sorbitol, including the alkoxylated derivatives of the polyols mentioned.

13. Product according to Claim 11, **characterized in that** said polyol is a polyol oligomer among polyether polyols, polyester polyols, and hydroxylated, optionally alkoxylated, acrylic oligomers, and in particular that it is present as a mixture with at least one other polyol a), preferably a polyol monomer as defined according to Claim 12.

14. Product according to one of Claims 1 to 13, **characterized in that**, in addition to said polyol a) having a functionality of at least 3, a second polyol different from the first having a functionality of at least 2 is present, this second polyol preferably being an oligoester diol, more preferentially comprising, as component in its repeat unit, said anhydride c) or its polyacid form.

15. Product according to one of Claims 1 to 14, **characterized in that** said polycarboxylic anhydride c) or its polyacid form or isomer of the latter is chosen from aromatic or cycloaliphatic or aliphatic polycarboxylic anhydrides or polycarboxylic acids, with the cycloaliphatic or aliphatic structures possibly being unsaturated.

16. Product according to Claim 15, **characterized in that** said polycarboxylic anhydride or polyacid or said isomer of the latter is aromatic and in particular selected from: (o-) phthalic anhydride, iso- or terephthalic acid, naphthenic anhydride or acid, trimellitic anhydride or acid, pyromellitic anhydride or pyromellitic acid, said anhydride or polyacid preferably having a functionality of 2.

17. Product according to Claim 15, **characterized in that** said anhydride or its polyacid form is selected from the corresponding cycloaliphatic anhydrides and diacids, in particular from tetrahydrophthalic anhydride and acid, dihydrophthalic anhydride and acid, nadic (bicyclo (2,2,1) hept-5-ene-2,3-dicarboxylic) anhydride and acid or cyclohexanedicarboxylic anhydride and acid.

18. Product according to Claim 15, **characterized in that** said anhydride or acid is aliphatic and in particular chosen from: maleic anhydride and acid, fumaric acid, itaconic anhydride and acid, and succinic anhydride and acid.

19. Product according to Claim 15, **characterized in that** said anhydride or acid is chosen from: (o-) phthalic anhydride, iso- or terephthalic acid, tetrahydrophthalic anhydride and acid, dihydrophthalic anhydride and acid, nadic anhydride and acid, maleic anhydride and acid, fumaric acid, itaconic anhydride and acid, and succinic anhydride and acid.

20. Product according to one of Claims 1 to 19, **characterized in that** said anhydride or polyacid is a mixture of at least two anhydrides and/or polyacids c).

21. Process for preparing a product as defined according to one of Claims 1 to 20, **characterized in that** it comprises:

i) mixing in a reactor of said polyol a) of the acrylic acid b), of said anhydride or of said polyacid c) in proportions such that the overall mole ratio $r = CO_2H/OH$ is less than 1, in particular greater than $[(m-1)*(r_1+1)]/[m*((r_1/2)+1)]$ and ranging up to 0.97, preferably greater than $1.05*[(m-1)*(r_1+1)]/[m*((r_1/2)+1)]$ and ranging up to 0.97 and more preferentially greater than $1.10*[(m-1)*(r_1+1)]/[m*((r_1/2)+1)]$ and ranging up to 0.97, with $r_1 = (CO_2H)_c/(CO_2H)_b$ being in the range of from 0.01 to 0.4, preferably from 0.05 to 0.35 and more preferentially from 0.1 to 0,3, more particularly for $z = 2$ and in the presence of an acidic esterification catalyst and of a solvent forming an azeotrope with water, to form the reaction mixture, followed by
ii) refluxing said reaction mixture, with simultaneous or successive and alternating reactions of esterification, by reaction of the acrylic acid b) and/or of said anhydride or polyacid c) with a hydroxyl of said polyol a) with formation of hydroxylated acrylate esters, and of etherification, via Michael addition reaction of a hydroxyl of said polyol or of said hydroxylated acrylate formed to an acrylate group and/or the acrylic acid b), and of esterification of the hydroxyl groups of said polyol and of said hydroxylated acrylates by said anhydride or diacid and gradual removal of the esterification water, with

iii) continuation of the reaction until complete consumption of the OH functions ($I_{OH}$ < 20 mg KOH/g) by Michael addition reactions and esterification reactions with said acrylic acid b) and said anhydride or diacid c),

iv) neutralization of said acidic catalyst before recovery of the final product, by removal of said solvent, without any other specific purification step required.

22. Crosslinkable composition, **characterized in that** it comprises at least one product as defined according to one of Claims 1 to 20 or obtained by means of a process as defined according to Claim 21.

23. Composition according to Claim 22, **characterized in that**, in the case where the calculated Mn of said product is greater than 1000, preferably greater than 1500, it comprises, in addition to said product, at least one reactive diluent, selected from acrylic monomers, preferably multifunctional acrylic monomers.

24. Composition according to Claim 22 or 23, **characterized in that** it a crosslinkable composition, preferably crosslinkable via radiation, in particular via UV radiation in the presence of a photoinitiating system or via an electron beam (EB) in the absence of a photoinitiating system and/or via a thermal radical initiating system, in particular via a peroxide initiating system (P-cure) and/or via Michael addition (M-cure) or via a mixed system, in particular via dual crosslinking (dual cure) and more particularly with the presence of at least two of the above-mentioned crosslinking systems.

25. Composition according to one of Claims 22 to 24, **characterized in that** it is a pigmented or non-pigmented coating composition, preferably chosen from paints, varnishes, inks or adhesives or a moulding composition or a sealing composition or a composite composition or a chemical sealing composition or a 3D printing composition or a composition for 3D objects produced layer-by-layer.

26. Use of a product as defined according to one of Claims 1 to 20 or obtained by means of a process as defined according to Claim 21, in crosslinkable compositions, in particular in crosslinkable compositions having a low degree of shrinkage, preferably in crosslinkable coating compositions.

27. Use according to Claim 26, **characterized in that** it applies to pigmented or non-pigmented coating compositions, preferably chosen from paints, varnishes, inks or adhesives or a moulding composition or a sealing composition or a composite composition or a chemical sealing composition or a 3D printing composition or a composition for 3D objects produced layer-by-layer.

28. Crosslinked final product, in particular chosen from crosslinked pigmented or non-pigmented coatings, preferably from paints, varnishes, inks or adhesives or chosen from moulded parts, seals, composites, chemical seals, 3D printing or 3D objects produced layer-by-layer, **characterized in that** it results from the use of at least one product as defined according to one of Claims 1 to 20 or obtained by means of a process as defined according to Claim 21 or from the crosslinking of a crosslinkable composition as defined according to one of Claims 22 to 25.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5096938 A
- FR 2401946
- JP 2010024380 B